# EUROPEAN PATENT SPECIFICATION

(11) **EP 3 830 299 B1**
(45) Date of publication and mention of the grant of the patent: **19.02.2025**
(21) Application number: 19753542.0
(22) Date of filing: 31.07.2019
(51) Int. Cl.: C12Q 1/6886

(54) **PREDICTION AND CHARACTERIZATION OF DLBCL CELL OF ORIGIN SUBTYPES**
VORHERSAGE UND CHARAKTERISIERUNG VON DLBCL-ZELLE VON URSPRUNGSSUBTYPEN
PRÉDICTION ET CARACTÉRISATION D'UNE CELLULE DLBCL DE SOUS-TYPES D'ORIGINE

(30) Priority: 01.08.2018 US 201862713434 P
(43) Date of publication of application: 09.06.2021
(73) Proprietor: GENENTECH, INC., South San Francisco, CA 94080 (US); Foundation Medicine, Inc., Cambridge, MA 02141 (US); F. Hoffmann-La Roche AG, 4070 Basel (CH)
(72) Inventor: TRABUCCO, Sally, Elizabeth, Cambridge, MA 02141 (US); BOLEN, Christopher, R., San Francisco, CA 94080-4990 (US); OESTERGAARD, Mikkel, Zahle, 4070 Basel (CH); SOKOL, Ethan, Samuel, Cambridge, MA 02141 (US); ALBACKER, Lee, Alan, Cambridge, MA 02141 (US)
(74) Representative: Mewburn Ellis LLP
(86) International application number: PCT/US2019/044497
(87) International publication number: WO 2020/028563

(56) References cited:
- WO-A1-2015/069790
- WO-A1-2016/090255
- WO-A1-2017/194670
- JORDINA ROVIRA ET AL: "MYD88 L265P Mutations, But No Other Variants, Identify a Subpopulation of DLBCL Patients of Activated B-cell Origin, Extranodal Involvement, and Poor Outcome", CLINICAL CANCER RESEARCH, vol. 22, no. 11, 20 January 2016 (2016-01-20), US, pages 2755 - 2764, XP055738136, ISSN: 1078-0432, DOI: 10.1158/1078-0432.CCR-15-1525
- ROLAND SCHMITZ ET AL: "Genetics and Pathogenesis of Diffuse Large B-Cell Lymphoma", THE NEW ENGLAND JOURNAL OF MEDICINE, vol. 378, no. 15, 12 April 2018 (2018-04-12), US, pages 1396 - 1407, XP055702698, ISSN: 0028-4793, DOI: 10.1056/NEJMoa1801445
- LYMPHOID NEOPLASIA ET AL: "Regular Article Genetic profiling of MYC and BCL2 in diffuse large B-cell lymphoma determines cell-of-origin?specific clinical impact", 28 March 2017 (2017-03-28), XP055738135, Retrieved from the Internet <URL:https://watermark.silverchair.com/blood747022.pdf?token=AQECAHi208BE49Ooan9kkhW_Ercy7Dm3ZL_9Cf3qfKAc485ysgAAA8QwggPABgkqhkiG9w0BBwagggOxMIIDrQIBADCCA6YGCSqGSIb3DQEHATAeBglghkgBZQMEAS4wEQQMVp49V6rl4jFF71i5AgEQgIIDd5wOp0YQ-NXjb7Km5Ir5TEKG8xeq1D5BZ-V84rpCXvTd8wvODWguSnd-mNrKMUREbCw6FJUuPAQyGOeKbWfOUkW3> [retrieved on 20201008], DOI: 10.1182/blood-2016-11-
- SCOTT DAVID W ET AL: "Dissecting aggressive B-cell lymphoma through genomic analysis - What is clinically relevant?", BEST PRACTICE & RESEARCH CLINICAL HAEMATOLOGY, vol. 31, no. 3, 7 July 2018 (2018-07-07), ELSEVIER, AMSTERDAM, NL, pages 187 - 198, XP085470250, ISSN: 1521-6926, DOI: 10.1016/J.BEHA.2018.07.003
- BOLEN CHRISTOPHER ET AL: "Systematic Analysis of the Prognostic Impact of Somatic Mutations in Diffuse Large B-Cell Lymphoma (DLBCL) with Evaluation of Cell-of-Origin Dependence: Results from the Phase 3 GOYA Trial in Previously Untreated DLBCL", vol. 130, SUPPL.1., 7 December 2017 (2017-12-07), XP009516970, Retrieved from the Internet <URL:https://ashpublications.org/blood/article/130/Supplement%201/2729/80419/Systematic-Analysis-of-the-Prognostic-Impact-of> [retrieved on 20191104], DOI: 10.1182/blood.V130.Suppl_1.2729.2729

## Description

### BACKGROUND OF THE INVENTION

Diffuse large B Cell lymphoma (DLBCL) is the most common form of non-Hodgkin's lymphoma, accounting for more than 25,000 new cases per year in the United States (R., T.L., et al. 2016 US lymphoid malignancy statistics by World Health Organization subtypes. CA: A Cancer Journal for Clinicians 66, 443-459 (2016)). Prognosis for patients with DLBCL is fairly good, with five-year survival rates between 55-62% (R., T.L., et al. 2016 US lymphoid malignancy statistics by World Health Organization subtypes. CA: A Cancer Journal for Clinicians 66, 443-459 (2016)). However, relapsed patients and those who do not initially respond to standard of care continue to have limited options, primarily consisting of autologous stem cell transplants or clinical trials. Additionally, although 60-65% of patients fully respond to R-CHOP (Rituximab, cyclophosphamide, doxorubicin, vincristine, and prednisone) therapy, there is a significant difference in response rates according to the DLBCL cell of origin (COO).

DLBCL has two COO subtypes: Activated B Cell (ABC) and Germinal Center B cell (GCB). The ABC subtype has a poor prognosis compared with GCB, and COO can be predictive for response to some new therapeutic agents. Traditionally, COO subtype has been determined by microarray assessed expression (ABC, GCB, unclassified), immunohistochemistry (IHC)-based algorithms (GCB or non-GCB), and expression based assays such as the Nanostring research use only lymphoma subtyping (LST) assay (ABC, GCB, unclassified) from Nanostring Technologies (also referred to herein as the Nanostring assay or Nanostring). Unfortunately, microarray is not typically feasible and some reports have failed to show a prognostic difference between GCB and non-GCB by IHC-based algorithms. This has led some to adopt the Nanostring assay as the preferred method to assess COO, but in some cases the tumor content or RNA quality is not sufficient to perform this assay. COO subtypes have differing gene mutations, with GCB typically characterized by *EZH2* alterations and *IGH:BCL2* translocations, while ABC is dominated by NF-KB and BCR signaling alterations such as *MYD88* and *CD79B.* Here we utilize mutational differences in COO subtypes to develop a COO DNA classification (COODC) model to predict COO from DNA-based features on a clinically utilized platform.

COO origin subtyping was originally developed using RNA expression microarrays (Alizadeh, A.A., et al. Distinct types of diffuse large B Cell lymphoma identified by gene expression profiling. Nature 403, 503 (2000)) and is a necessary component of DLBCL clinical care per the 2016 updated WHO lymphoid neoplasm classifications (Swerdlow, S.H., et al. The 2016 revision of the World Health Organization classification of lymphoid neoplasms. Blood 127, 2375-2390 (2016)). RNA-based classifiers commonly split DLBCL into the activated B Cell (ABC) and germinal center (GC) B Cell (GCB) subtypes. DLBCL that does not fall into either of these categories are called unclassified. These subtypes are both prognostic and predictive. Among patients treated with modern anti-CD20 therapies, the ABC subtype has a worse prognosis, with a three-year progression free survival of 59% compared with 75% for GCB patients (Vitolo, U., et al. Obinutuzumab or Rituximab Plus Cyclophosphamide, Doxorubicin, Vincristine, and Prednisone in Previously Untreated Diffuse Large B Cell Lymphoma. Journal of Clinical Oncology 35, 3529-3537 (2017)). However, targeted therapeutics, including the BCR signaling inhibitor ibrutinib, have recently been developed that target pathways commonly altered in ABC. Ibrutinib has shown significant clinical benefit among patients with mutations in BCR signaling genes, which are common in the ABC subset (Wilson, W.H., et al. Targeting B cell receptor signaling with ibrutinib in diffuse large B cell lymphoma. Nature Medicine 21, 922 (2015)). It is clear that accurate assessment of COO subtype is critical for the appropriate clinical management of DLBCL.

In most clinical settings it is not feasible to perform microarray analyses for each patient. This has led to the development and adoption of immunohistochemistry (IHC) based algorithms to approximate COO. The most widely used of these is the Hans' algorithm (Hans, C.P., et al. Confirmation of the molecular classification of diffuse large B Cell lymphoma by immunohistochemistry using a tissue microarray. Blood 103, 275-282 (2003)), which is able to classify GCB and non-GC DLBCL, the latter of which includes both ABC and unclassified subtypes. Unfortunately, these algorithms do not reliably recapitulate the original prognostic findings, and in some cases IHC based algorithms show no significant survival difference between GCB and non-GCB types (Gribben, R.C., et al. Poor Concordance among Nine Immunohistochemistry Classifiers of Cell-of-Origin for Diffuse Large B Cell Lymphoma: Implications for Therapeutic Strategies. (2013); Meyer, P.N., et al. Immunohistochemical Methods for Predicting Cell of Origin and Survival in Patients With Diffuse Large B Cell Lymphoma Treated With Rituximab. Journal of Clinical Oncology 29, 200-207 (2011)).

Given the need for reliable, clinically useful methods to determine COO for DLBCL, recent efforts have focused on simpler, more streamlined methods, including the Nanostring assay (Scott, D.W., et al. Determining cell-of-origin subtypes of diffuse large B Cell lymphoma using gene expression in formalin-fixed paraffin-embedded tissue. Blood 123, 1214-1217 (2014)). This method uses RNA expression from 20 genes to determine COO with 98% concordance to microarray and 8.8% unclassified calls compared with 14.7% by the gold standard microarray. While this holds great promise for clinical utility, it requires RNA and samples with at least 60% tumor purity (i.e., tumor content) to achieve this accuracy (Scott, D.W., et al. Determining cell-of-origin subtypes of diffuse large B Cell lymphoma using gene expression in formalin-fixed paraffin-embedded tissue. Blood 123, 1214-1217 (2014)), which is not routinely accessible.

COO subtypes have differing gene mutations, with GCB typically characterized by *EZH2* alterations and *IGH:BCL2* translocations, while ABC is dominated by NF-KB and BCR signaling alterations such as *MYD88* and *CD79B.* Chapuy and colleagues recently showed distinct mutational signatures in DLBCL samples, including a canonical activation-induced cytidine deaminase (AID) signature (Chapuy, B., et al. Molecular subtypes of diffuse large B cell lymphoma are associated with distinct pathogenic mechanisms and outcomes. Nature Medicine 24, 679-690 (2018)). Although previous reports do not identify mutational signature differences between subtypes, it is hypothesized that there are differences in mutational profiles of GCB and ABC tumors and these are most likely the result of distinct histories of precursor cells. GCB DLBCLs are thought to arise from germinal center (GC) B Cells, while ABC subtype is thought to arise from post-GC B Cells. During the antibody maturation process, GC B Cells are subject to class switch recombination (CSR) and somatic hypermutation (SHM) induced by AID protein that induces double strand breaks (DSB) and single base mutations. Recent studies have shown certain genes in DLBCL tumors have evidence of frequent AID induced alterations (Chapuy, B., et al. Molecular subtypes of diffuse large B cell lymphoma are associated with distinct pathogenic mechanisms and outcomes. Nature Medicine 24, 679-690 (2018); Schmitz, R., et al. Genetics and Pathogenesis of Diffuse Large B Cell Lymphoma. New England Journal of Medicine 378, 1396-1407 (2018)) and certain DLBCL-related genes, such as PIM1, are thought to be hotspots for off-target AID activity.

In order to solve the problem of finding a reliable diagnostic for a DLBCL COO that does not require RNA and does not require samples with at least 60% tumor purity, a DNA-only classifier using Foundation Medicine's FoundationOne^{®}Heme platform was invented and is disclosed herein. It is shown that this assay maintains 89% (e.g., approximately 90%) concordance to Nanostring, and requires samples having only 20% tumor content (e.g., approximately 20% tumor content). Furthermore, it is shown that the COO classifications from this assay reliably recapitulate the prognostic differences observed in RNA-based classifications like Nanostring.

### BRIEF SUMMARY OF THE INVENTION

A DNA-based method to determine COO for DLBCL was invented and is disclosed herein. In certain embodiments, this assay maintains 89% concordance to Nanostring. In certain embodiments, this assay requires samples having 20% tumor content as compared to the 60% required for Nanostring.

In some embodiments, provided herein is a method of determining the cell of origin (COO) of diffuse large B Cell lymphoma (DLBCL) using a cell of origin DNA classification (COODC). In certain embodiments according to (or as applied to) any of the embodiments above, the COODC comprises: (a) acquiring, e.g., collecting, a sample, e.g., a clinical sample, from a patient diagnosed with DLBCL, (b) performing DNA sequencing on the sample, e.g., clinical sample, and (c) applying a pre-defined COODC classifier to a list of genomic features (e.g., one or more features described in Table 1) to calculate a predictor score. In certain embodiments according to (or as applied to) any of the embodiments above, the DNA sequencing is performed using the DNA component of the FoundationOne^{®}Heme platform. In certain embodiments according to (or as applied to) any of the embodiments above, the DNA sequencing comprises targeted DNA-sequencing of approximately 300-500 genes. In certain embodiments according to (or as applied to) any of the embodiments above, the detection of alterations in the BCL2, EZH2, and TNSFRSF14 genes using the COODC predicts the COO of the DLBCL is a germinal center B Cell (GCB). In certain embodiments according to (or as applied to) any of the embodiments above, alterations in the BCL2, EZH2, and TNSFRSF14 genes are detected. In certain embodiments according to (or as applied to) any of the embodiments above, the detection of alterations in the chromosome 3q copy number, MYD88, and CD79B genes using the COODC predicts the COO of the DLBCL is an activated B Cell (ABC). In certain embodiments according to (or as applied to) any of the embodiments above, alterations in the chromosome 3q copy number, MYD88, and CD79B genes are detected. In certain embodiments according to (or as applied to) any of the embodiments above, the detection of alterations in the NOTCH1, NOTCH2, and BCL6 genes using the COODC predicts the COO of the DLBCL is an activated B Cell (ABC). In certain embodiments according to (or as applied to) any of the embodiments above, alterations in the NOTCH1, NOTCH2, and BCL6 genes are detected. In certain embodiments according to (or as applied to) any of the embodiments above, the detection of IGH:BCL2 rearrangements, CREBBP alterations, and TNFRSF14 alterations predicts the COO of the DLBCL is a GCB. In certain embodiments according to (or as applied to) any of the embodiments above, IGH:BCL2 rearrangements, CREBBP alterations, and TNFRSF14 alterations are detected. In certain embodiments according to (or as applied to) any of the embodiments above, the detection of CDKN2A/B deletions, CD79B alterations at amino acid 196, and MYD88 alterations at amino acid 265 predicts the COO of the DLBCL is an ABC. In certain embodiments according to (or as applied to) any of the embodiments above, a CDKN2A/B deletion, a CD79B alteration at amino acid 196, and an MYD88 alteration at amino acid 265 are detected. In certain embodiments according to (or as applied to) any of the embodiments above, a COSMIC signature 3 predicts the COO of the DLBCL is GCB. In certain embodiments according to (or as applied to) any of the embodiments above, a COSMIC signature 3 is detected. In certain embodiments according to (or as applied to) any of the embodiments above, the method further comprises the step of (d) classifying a patient having a predictor score below a low cutoff (e.g., a low pre-defined cutoff) as GCB, and a patient having a predictor score above a high cutoff (e.g., a high pre-defined cutoff) as ABC. In certain embodiments according to (or as applied to) any of the embodiments above, the method further comprises the step of (e) classifying a patient having a predictor score above or equal to the low cutoff (e.g., the low pre-defined cutoff) and below the high cutoff (e.g., the high pre-defined cutoff) as unclassified. In certain embodiments according to (or as applied to) any of the embodiments above, the COODC was developed using the methods described in Examples 3-9. In certain embodiments according to (or as applied to) any of the embodiments above, no RNA analysis is performed. In certain embodiments according to (or as applied to) any of the embodiments above, RNA analysis does not contribute substantially to the determination of the COO. In certain embodiments according to (or as applied to) any of the embodiments above, no immunohistochemistry (IHC) analysis is performed. In certain embodiments according to (or as applied to) any of the embodiments above, IHC analysis does not contribute substantially to the determination of the COO.

In some embodiments, provided herein is a method of determining whether the cell of origin (COO) of diffuse large B Cell lymphoma (DLBCL) is an activated B Cell (ABC) or a germinal center B Cell (GCB) using a cell of origin DNA classification (COODC) model. In certain embodiments according to (or as applied to) any of the embodiments above, the method comprises (a) acquiring, e.g., collecting, a sample, e.g., a clinical sample, of a patient diagnosed with DLBCL, (b) performing DNA sequencing on the sample, e.g., clinical sample, (c) applying the pre-defined COODC classifier to the list of genomic features (e.g., one or more features described in Table 1) to calculate a predictor score, and (d) classifying a patient having a predictor score below a low cutoff (e.g., a low pre-defined cutoff) as GCB, and a patient having a predictor score above or equal to a high cutoff (e.g., a high pre-defined cutoff) as ABC. In certain embodiments according to (or as applied to) any of the embodiments above, the method further comprises the step of (e) classifying a patient having a predictor score above or equal to the low cutoff (e.g., the low pre-defined cutoff) and below the high cutoff (e.g., the high pre-defined cutoff) as unclassified. In certain embodiments according to (or as applied to) any of the embodiments above, the DNA sequencing is performed using the DNA component of the FoundationOne^{®}Heme platform. In certain embodiments according to (or as applied to) any of the embodiments above, the DNA sequencing comprises targeted DNA-sequencing of approximately 300-500 genes. In certain embodiments according to (or as applied to) any of the embodiments above, the detection of alterations in the BCL2, EZH2, and TNSFRSF14 genes using the COODC predicts the COO of the DLBCL is germinal center B Cell (GCB). In certain embodiments according to (or as applied to) any of the embodiments above, alterations in the BCL2, EZH2, and TNSFRSF14 genes are detected. In certain embodiments according to (or as applied to) any of the embodiments above, the detection of alterations in the chromosome 3q copy number, MYD88, and CD79B genes using the COODC predicts the COO of the DLBCL is an activated B Cell (ABC). In certain embodiments according to (or as applied to) any of the embodiments above, alterations in the chromosome 3q copy number, MYD88, and CD79B genes are detected. In certain embodiments according to (or as applied to) any of the embodiments above, the detection of alterations in the NOTCH1, NOTCH2, and BCL6 genes using the COODC predicts the COO of the DLBCL is an activated B Cell (ABC). In certain embodiments according to (or as applied to) any of the embodiments above, of alterations in the NOTCH1, NOTCH2, and BCL6 genes are detected. In certain embodiments according to (or as applied to) any of the embodiments above, the detection of IGH:BCL2 rearrangements, CREBBP alterations, and TNFRSF14 alterations predicts the COO of the DLBCL is GCB. In certain embodiments according to (or as applied to) any of the embodiments above, IGH:BCL2 rearrangements, CREBBP alterations, and TNFRSF14 alterations are detected. In certain embodiments according to (or as applied to) any of the embodiments above, the detection of CDKN2A/B deletions, CD79B alterations at amino acid 196, and MYD88 alterations at amino acid 265 predicts the COO of the DLBCL is ABC. In certain embodiments according to (or as applied to) any of the embodiments above, CDKN2A/B deletions, CD79B alterations at amino acid 196, and MYD88 alterations at amino acid 265 are detected. In certain embodiments according to (or as applied to) any of the embodiments above, a COSMIC signature 3 predicts the COO of the DLBCL is GCB. In certain embodiments according to (or as applied to) any of the embodiments above, a COSMIC signature 3 is detected. In certain embodiments according to (or as applied to) any of the embodiments above, the COODC was developed using the methods described in Examples 3-9. In certain embodiments according to (or as applied to) any of the embodiments above, no RNA analysis is performed. In certain embodiments according to (or as applied to) any of the embodiments above, RNA analysis does not contribute substantially to the determination of COO. In certain embodiments according to (or as applied to) any of the embodiments above, no immunohistochemistry (IHC) analysis is performed. In certain embodiments according to (or as applied to) any of the embodiments above, IHC does not contribute substantially to the determination of COO.

In some embodiments, provided herein is a method of designing a therapy for the treatment of diffuse large B Cell lymphoma (DLBCL) using a cell of origin DNA classification (COODC) model comprising: (a) acquiring, e.g., collecting, a sample, e.g., a clinical sample, of a patient diagnosed with DLBCL, (b) performing DNA sequencing on the sample, e.g., clinical sample, (c) applying the pre-defined COODC classifier to the list of genomic features (e.g., one or more features described in Table 1) to calculate a predictor score, and (d) classifying a patient having a predictor score below a low cutoff (e.g., a low pre-defined cutoff) as GCB, and a patient having a predictor score above or equal to a high cutoff (e.g., a high pre-defined cutoff) as ABC, wherein the patient is administered a therapy recommended for their COO. In certain embodiments according to (or as applied to) any of the embodiments above, the method further comprises the step of (e) classifying a patient having a predictor score above or equal to the low cutoff (e.g., the low pre-defined cutoff) and below the high cutoff (e.g., the high pre-defined cutoff) as unclassified. In certain embodiments according to (or as applied to) any of the embodiments above, the GCB COO patient is administered a therapy that is effective for GCB COO. In certain embodiments according to (or as applied to) any of the embodiments above, the ABC COO patient is administered a therapy that is effective for ABC COO.

In some embodiments, provided herein is a method of predicting response to therapy for the treatment of diffuse large B Cell lymphoma (DLBCL) using a cell of origin DNA classification (COODC) model comprising (a) acquiring, e.g., collecting, a sample, e.g., a clinical sample, of a patient diagnosed with DLBCL, (b) performing DNA sequencing on the sample, e.g., clinical sample, (c) applying the pre-defined COODC classifier to the list of genomic features (e.g., one or more features described in Table 1) to calculate a predictor score, and (d) classifying a patient having a predictor score below a low cutoff (e.g., a low pre-defined cutoff) as GCB, and a patient having a predictor score above or equal to a high cutoff (e.g., a high pre-defined cutoff) as ABC, wherein the response to a therapy for the treatment of DLBCL is dependent on the COO. In certain embodiments according to (or as applied to) any of the embodiments above, the method further comprises the step of (e) classifying a patient having a predictor score above or equal to the low cutoff (e.g., the low pre-defined cutoff) and below the high cutoff (e.g., the high pre-defined cutoff) as unclassified. In certain embodiments according to (or as applied to) any of the embodiments above, the ABC COO is predictive for response to therapies known to be effective on the ABC subtype. In certain embodiments according to (or as applied to) any of the embodiments above, the therapy comprises ibrutinib and/or lenalidomide. In certain embodiments according to (or as applied to) any of the embodiments above, the GCB COO is predictive for response to therapies known to be effective on the GCB subtype. In certain embodiments according to (or as applied to) any of the embodiments above, the therapy comprises ibrutinib and/or lenalidomide. In certain embodiments according to (or as applied to) any of the embodiments above, the cell of origin (COO) of diffuse large B Cell lymphoma (DLBCL) is determined using a DNA-based platform method wherein neither RNA (e.g., analysis of RNA) nor immunohistochemistry is a component of the method. In certain embodiments according to (or as applied to) any of the embodiments above, the DNA-based platform is the COODC described in any of the embodiments described herein.

In some embodiments, provided herein is a cell of origin DNA classification (COODC) diagnostic used to determine whether the cell of origin (COO) of diffuse large B Cell lymphoma (DLBCL) is an activated B Cell (ABC) or a germinal center B Cell (GCB). In certain embodiments according to (or as applied to) any of the embodiments above, the COODC is the COODC described in any of the embodiments described herein. In certain embodiments according to (or as applied to) any of the embodiments above, the COODC is a kit.

In some embodiments, provided herein is a method for using a cell of origin DNA classification (COODC) diagnostic to determine whether the cell of origin (COO) of diffuse large B Cell lymphoma (DLBCL) is an activated B Cell (ABC) or a germinal center B Cell (GCB) comprising: (a) acquiring, e.g., collecting, a sample, e.g., a clinical sample, of a patient diagnosed with DLBCL, (b) performing DNA sequencing on the sample, e.g., clinical sample, (c) applying a COODC classifier (e.g., a pre-defined COODC classifier) to the list of genomic features (e.g., one or more features described in Table 1) to calculate a predictor score, and (d) classifying a patient having a predictor score below a low cutoff (e.g., a low pre-defined cutoff) as GCB, and a patient having a predictor score above or equal to a high cutoff (e.g., a high pre-defined cutoff) as ABC. In certain embodiments according to (or as applied to) any of the embodiments above, the method further comprises the step of (e) classifying a patient having a predictor score above or equal to the low cutoff (e.g., the low pre-defined cutoff) and below the high cutoff (e.g., the high pre-defined cutoff) as unclassified. In certain embodiments according to (or as applied to) any of the embodiments above, the diagnostic is a kit. In certain embodiments according to (or as applied to) any of the embodiments above, the COO is determined to be ABC or GCB according to any of any of the embodiments above.

In certain embodiments according to (or as applied to) any of the embodiments above, the sample is a clinical sample. In certain embodiments according to (or as applied to) any of the embodiments above, the clinical sample is tumor biopsy, blood, bone marrow aspirate, or an extracted nucleic acid. In certain embodiments according to (or as applied to) any of the embodiments above, the tumor biopsy is prepared on a formalin-fixed paraffin-embedded (FFPE) slide. In certain embodiments according to (or as applied to) any of the embodiments above, the DNA sequencing comprises targeted DNA-sequencing of approximately 465 genes. In certain embodiments according to (or as applied to) any of the embodiments above, a plurality of samples (e.g., a plurality of clinical samples), e.g., from a plurality of patients, are acquired (e.g., collected).

In some embodiments, provided herein is a method of selecting, classifying, or treating a subject comprising acquiring or providing a value for cell of origin (COO) and in response to the value selecting, classifying, evaluating, or treating the subject. In certain embodiments according to (or as applied to) any of the embodiments above, the value is acquired from another entity, e.g., a laboratory. In certain embodiments according to (or as applied to) any of the embodiments above, the value is substantially the same as the value that would be provided by the method or diagnostic of any of the embodiments described herein. In certain embodiments according to (or as applied to) any of the embodiments above, the value is determined by any of the embodiments described herein. In certain embodiments according to (or as applied to) any of the embodiments above, the subject has DLBCL.

In certain embodiments according to (or as applied to) any of the embodiments above, the low cutoff (e.g., the low pre-defined cutoff) for classifying patents or samples as GCB is between 0.2-0.3, e.g., between 0.21-0.29, 0.22-0.28, 0.23-0.27, 0.24-0.26, 0.21-0.3, 0.22-0.3, 0.23-0.3, 0.24-0.3, 0.25-0.3, 0.26-0.3, 0.27-0.3, 0.28-0.3, 0.29-0.3, 0.2-0.29, 0.2-0.28, 0.2-0.27, 0.2-0.26, 0.2-0.25, 0.2-0.24, 0.2-0.23, 0.2-0.22, or 0.2-0.21, e.g., 0.200, 0.201, 0.202, 0.203, 0.204, 0.205, 0.206, 0.207, 0.208, 0.209, 0.210, 0.211, 0.212, 0.213, 0.214, 0.215, 0.216, 0.217, 0.218, 0.219, 0.220, 0.221, 0.222, 0.223, 0.224, 0.225, 0.226, 0.227, 0.228, 0.229, 0.230, 0.231, 0.232, 0.233, 0.234, 0.235, 0.236, 0.237, 0.238, 0.239, 0.240, 0.241, 0.242, 0.243, 0.244, 0.245, 0.246, 0.247, 0.248, 0.249, 0.250, 0.251, 0.252, 0.253, 0.254, 0.255, 0.256, 0.257, 0.258, 0.259, 0.260, 0.261, 0.262, 0.263, 0.264, 0.265, 0.266, 0.267, 0.268, 0.269, 0.270, 0.271, 0.272, 0.273, 0.274, 0.275, 0.276, 0.277, 0.278, 0.279, 0.280, 0.281, 0.282, 0.283, 0.284, 0.285, 0.286, 0.287, 0.288, 0.289, 0.290, 0.291, 0.292, 0.293, 0.294, 0.295, 0.296, 0.297, 0.298, 0.299 or 0.300. In certain embodiments according to (or as applied to) any of the embodiments above, the high cutoff (e.g., the high pre-defined cutoff) for classifying samples as ABC is between 0.4-0.6, e.g., 0.41-0.59, 0.42-0.58, 0.43-0.57, 0.44-0.56, 0.45-0.55, 0.46-0.54, 0.47-0.53, 0.48-0.52, 0.49-0.51, 0.4-0.59, 0.4-0.58, 0.4-0.57, 0.4-0.56, 0.4-0.55, 0.4-0.54, 0.4-0.53, 0.4-0.52, 0.4-0.51, 0.4-0.50. 0.4-0.49, 0.4-0.48, 0.4-0.47, 0.4-0.46, 0.4-0.45, 0.4-0.44, 0.4-0.42, 0.4-0.41, 0.41-0.6, 0.42-0.6, 0.43-0.6, 0.44-0.6, 0.45-0.6, 0.46-0.6, 0.47-0.6, 0.48-0.6, 0.49-0.6, 0.5-0.6, 0.51-0.6, 0.52-0.6, 0.53-0.6, 0.54-0.6, 0.55-0.6, 0.56-0.6, 0.57-0.6, 0.58-0.6, or 0.59-0.6, e.g., 0.400, 0.401, 0.402, 0.403, 0.404, 0.405, 0.406, 0.407, 0.408, 0.409, 0.410, 0.411, 0.412, 0.413, 0.414, 0.415, 0.416, 0.417, 0.418, 0.419, 0.420, 0.421, 0.422, 0.423, 0.424, 0.425, 0.426, 0.427, 0.428, 0.429, 0.430, 0.431, 0.432, 0.433, 0.434, 0.435, 0.436, 0.437, 0.438, 0.439, 0.440, 0.441, 0.442, 0.443, 0.444, 0.445, 0.446, 0.447, 0.448, 0.449, 0.450, 0.451, 0.452, 0.453, 0.454, 0.455, 0.456, 0.457, 0.458, 0.459, 0.460, 0.461, 0.462, 0.463, 0.464, 0.465, 0.466, 0.467, 0.468, 0.469, 0.470, 0.471, 0.472, 0.473, 0.474, 0.475, 0.476, 0.477, 0.478, 0.479, 0.480, 0.481, 0.482, 0.483, 0.484, 0.485, 0.486, 0.487, 0.488, 0.489, 0.490, 0.491, 0.492, 0.493, 0.494, 0.495, 0.496, 0.497, 0.498, 0.499, 0.500, 0.501, 0.502, 0.503, 0.504, 0.505, 0.506, 0.507, 0.508, 0.509, 0.510, 0.511, 0.512, 0.513, 0.514, 0.515, 0.516, 0.517, 0.518, 0.519, 0.520, 0.521, 0.522, 0.523, 0.524, 0.525, 0.526, 0.527, 0.528, 0.529, 0.530, 0.531, 0.532, 0.533, 0.534, 0.535, 0.536, 0.537, 0.538, 0.539, 0.540, 0.541, 0.542, 0.543, 0.544, 0.545, 0.546, 0.547, 0.548, 0.549, 0.550, 0.551, 0.552, 0.553, 0.554, 0.555, 0.556, 0.557, 0.558, 0.559, 0.560, 0.561, 0.562, 0.563, 0.564, 0.565, 0.566, 0.567, 0.568, 0.569, 0.570, 0.571, 0.572, 0.573, 0.574, 0.575, 0.576, 0.577, 0.578, 0.579, 0.580, 0.581, 0.582, 0.583, 0.584, 0.585, 0.586, 0.587, 0.588, 0.589, 0.590, 0.591, 0.592, 0.593, 0.594, 0.595, 0.596, 0.597, 0.598, 0.599 or 0.600.

In certain embodiments according to (or as applied to) any of the embodiments above, if the predictor score is less than 0.3, e.g., less than 0.299, 0.298, 0.297, 0.296, 0.295, 0.294, 0.293, 0.292, 0.291, 0.29, 0.289, 0.288, 0.287, 0.286, 0.285, 0.284, 0.283, 0.282, 0.281, 0.28, 0.279, 0.278, 0.277, 0.276, 0.275, 0.274, 0.273, 0.272, 0.271, 0.27, 0.269, 0.268, 0.267, 0.266, 0.265, 0.264, 0.263, 0.262, 0.261, 0.26, 0.259, 0.258, 0.257, 0.256, 0.255, 0.254, 0.253, 0.252, 0.251, 0.25, 0.249, 0.248, 0.247, 0.246, 0.245, 0.244, 0.243, 0.242, 0.241, 0.24, 0.239, 0.238, 0.237, 0.236, 0.235, 0.234, 0.233, 0.232, 0.231, 0.23, 0.229, 0.228, 0.227, 0.226, 0.225, 0.224, 0.223, 0.222, 0.221, 0.22, 0.219, 0.218, 0.217, 0.216, 0.215, 0.214, 0.213, 0.212, 0.211, 0.21, 0.209, 0.208, 0.207, 0.206, 0.205, 0.204, 0.203, 0.202, 0.201, 0.200, 0.19, 0.18, 0.17, 0.16, 0.15, 0,14, 0.13, 0.12, 0.11, 0.1, 0.09, 0.08, 0.07, 0.06, 0.05, 0.04, 0.03, 0.02, or 0.01, the patient or sample is classified as GCB. In certain embodiments according to (or as applied to) any of the embodiments above, if the predictor score is greater than or equal to 0.4, e.g., greater than or equal to 0.401, 0.402, 0.403, 0.404, 0.405, 0.406, 0.407, 0.408, 0.409, 0.410, 0.411, 0.412, 0.413, 0.414, 0.415, 0.416, 0.417, 0.418, 0.419, 0.420, 0.421, 0.422, 0.423, 0.424, 0.425, 0.426, 0.427, 0.428, 0.429, 0.430, 0.431, 0.432, 0.433, 0.434, 0.435, 0.436, 0.437, 0.438, 0.439, 0.440, 0.441, 0.442, 0.443, 0.444, 0.445, 0.446, 0.447, 0.448, 0.449, 0.450, 0.451, 0.452, 0.453, 0.454, 0.455, 0.456, 0.457, 0.458, 0.459, 0.460, 0.461, 0.462, 0.463, 0.464, 0.465, 0.466, 0.467, 0.468, 0.469, 0.470, 0.471, 0.472, 0.473, 0.474, 0.475, 0.476, 0.477, 0.478, 0.479, 0.480, 0.481, 0.482, 0.483, 0.484, 0.485, 0.486, 0.487, 0.488, 0.489, 0.490, 0.491, 0.492, 0.493, 0.494, 0.495, 0.496, 0.497, 0.498, 0.499, 0.500, 0.501, 0.502, 0.503, 0.504, 0.505, 0.506, 0.507, 0.508, 0.509, 0.510, 0.511, 0.512, 0.513, 0.514, 0.515, 0.516, 0.517, 0.518, 0.519, 0.520, 0.521, 0.522, 0.523, 0.524, 0.525, 0.526, 0.527, 0.528, 0.529, 0.530, 0.531, 0.532, 0.533, 0.534, 0.535, 0.536, 0.537, 0.538, 0.539, 0.540, 0.541, 0.542, 0.543, 0.544, 0.545, 0.546, 0.547, 0.548, 0.549, 0.550, 0.551, 0.552, 0.553, 0.554, 0.555, 0.556, 0.557, 0.558, 0.559, 0.560, 0.561, 0.562, 0.563, 0.564, 0.565, 0.566, 0.567, 0.568, 0.569, 0.570, 0.571, 0.572, 0.573, 0.574, 0.575, 0.576, 0.577, 0.578, 0.579, 0.580, 0.581, 0.582, 0.583, 0.584, 0.585, 0.586, 0.587, 0.588, 0.589, 0.590, 0.591, 0.592, 0.593, 0.594, 0.595, 0.596, 0.597, 0.598, 0.599, 0.60, 0.61, 0.62, 0.63, 0.64, 0.66, 0.66, 0.67, 0.68, 0.69, 0.70, 0.71, 0.72, 0.73, 0.74, 0.77, 0.76, 0.77, 0.78, 0.79, 0.80, 0.81, 0.82, 0.83, 0.84, 0.88, 0.86, 0.87, 0.88, 0.89, 0.90, 0.90, 0.91, 0.92, 0.93, 0.94, 0.99, 0.96, 0.97, 0.98, or 0.99, the patient or sample is classified as ABC.

In certain embodiments according to (or as applied to) any of the embodiments above, if the predictor score is less than the high pre-defined cutoff, but greater than or equal to the low pre-defined cutoff, then the patient or sample is unclassified. In certain embodiments according to (or as applied to) any of the embodiments above, if the predictor score is greater than or equal to a low pre-defined cutoff described herein (e.g., between 0.2-0.3) and less than a high pre-defined cutoff described herein (e.g., between 0.4-0.6), e.g., greater than or equal to (a) any one of 0.299, 0.298, 0.297, 0.296, 0.295, 0.294, 0.293, 0.292, 0.291, 0.29, 0.289, 0.288, 0.287, 0.286, 0.285, 0.284, 0.283, 0.282, 0.281, 0.28, 0.279, 0.278, 0.277, 0.276, 0.275, 0.274, 0.273, 0.272, 0.271, 0.27, 0.269, 0.268, 0.267, 0.266, 0.265, 0.264, 0.263, 0.262, 0.261, 0.26, 0.259, 0.258, 0.257, 0.256, 0.255, 0.254, 0.253, 0.252, 0.251, 0.25, 0.249, 0.248, 0.247, 0.246, 0.245, 0.244, 0.243, 0.242, 0.241, 0.24, 0.239, 0.238, 0.237, 0.236, 0.235, 0.234, 0.233, 0.232, 0.231, 0.23, 0.229, 0.228, 0.227, 0.226, 0.225, 0.224, 0.223, 0.222, 0.221, 0.22, 0.219, 0.218, 0.217, 0.216, 0.215, 0.214, 0.213, 0.212, 0.211, 0.21, 0.209, 0.208, 0.207, 0.206, 0.205, 0.204, 0.203, 0.202, 0.201 or 0.200 and (b) less than any one of 0.401, 0.402, 0.403, 0.404, 0.405, 0.406, 0.407, 0.408, 0.409, 0.410, 0.411, 0.412, 0.413, 0.414, 0.415, 0.416, 0.417, 0.418, 0.419, 0.420, 0.421, 0.422, 0.423, 0.424, 0.425, 0.426, 0.427, 0.428, 0.429, 0.430, 0.431, 0.432, 0.433, 0.434, 0.435, 0.436, 0.437, 0.438, 0.439, 0.440, 0.441, 0.442, 0.443, 0.444, 0.445, 0.446, 0.447, 0.448, 0.449, 0.450, 0.451, 0.452, 0.453, 0.454, 0.455, 0.456, 0.457, 0.458, 0.459, 0.460, 0.461, 0.462, 0.463, 0.464, 0.465, 0.466, 0.467, 0.468, 0.469, 0.470, 0.471, 0.472, 0.473, 0.474, 0.475, 0.476, 0.477, 0.478, 0.479, 0.480, 0.481, 0.482, 0.483, 0.484, 0.485, 0.486, 0.487, 0.488, 0.489, 0.490, 0.491, 0.492, 0.493, 0.494, 0.495, 0.496, 0.497, 0.498, 0.499, 0.500, 0.501, 0.502, 0.503, 0.504, 0.505, 0.506, 0.507, 0.508, 0.509, 0.510, 0.511, 0.512, 0.513, 0.514, 0.515, 0.516, 0.517, 0.518, 0.519, 0.520, 0.521, 0.522, 0.523, 0.524, 0.525, 0.526, 0.527, 0.528, 0.529, 0.530, 0.531, 0.532, 0.533, 0.534, 0.535, 0.536, 0.537, 0.538, 0.539, 0.540, 0.541, 0.542, 0.543, 0.544, 0.545, 0.546, 0.547, 0.548, 0.549, 0.550, 0.551, 0.552, 0.553, 0.554, 0.555, 0.556, 0.557, 0.558, 0.559, 0.560, 0.561, 0.562, 0.563, 0.564, 0.565, 0.566, 0.567, 0.568, 0.569, 0.570, 0.571, 0.572, 0.573, 0.574, 0.575, 0.576, 0.577, 0.578, 0.579, 0.580, 0.581, 0.582, 0.583, 0.584, 0.585, 0.586, 0.587, 0.588, 0.589, 0.590, 0.591, 0.592, 0.593, 0.594, 0.595, 0.596, 0.597, 0.598, 0.599 or 0.60, e.g., if the predictor score is between 0.2-0.6, 0.22-0.58, 0.24-0.56, 0.26-0.54, 0.28-0.52, 0.3-0.5, 0.32-0.48, 0.34-0.46, 0.36-0.44, 0.38-0.42, 0.2-0.4, 0.22-0.38, 0.24-0.36, 0.26-0.34, 0.28-0.32, 0.3-0.6, 0.32-0.58, 0.34-0.56, 0.36-0.54, 0.38-0.52, 0.4-0.5, 0.42-0.48, or 0.44-0.46, e.g., between 0.3-0.4, 0.31-0.39, 0.32-0.38, 0.33-0.37, 0.34-0.36, 0.3-0.39, 0.3-0.38, 0.3-0.37, 0.3-0.36, 0.3-0.35, 0.3-0.34, 0.3-0.33, 0.3-0.32, 0.3-0.31, 0.31-0.4, 0.32-0.4, 0.33-0.4, 0.34-0.4, 0.35-0.4, 0.36-0.4, 0.37-0.4, 0.38-0.4, 0.39-0.4, e.g., 0.3, 0.301, 0.302, 0.303, 0.304, 0.305, 0.306, 0.307, 0.308, 0.309, 0.31, 0.311, 0.312, 0.313, 0.314, 0.315, 0.316, 0.317, 0.318, 0.319, 0.32, 0.321, 0.322, 0.323, 0.324, 0.325, 0.326, 0.327, 0.328, 0.329, 0.33, 0.331, 0.332, 0.333, 0.334, 0.335, 0.336, 0.337, 0.338, 0.339, 0.34, 0.341, 0.342, 0.343, 0.344, 0.345, 0.346, 0.347, 0.348, 0.349, 0.35, 0.351, 0.352, 0.353, 0.354, 0.355, 0.356, 0.357, 0.358, 0.359, 0.36, 0.361, 0.362, 0.363, 0.364, 0.365, 0.366, 0.367, 0.368, 0.369, 0.37, 0.371, 0.372, 0.373, 0.374, 0.375, 0.376, 0.377, 0.378, 0.379, 0.38, 0.381, 0.382, 0.383, 0.384, 0.385, 0.386, 0.387, 0.388, 0.389, 0.39, 0.391, 0.392, 0.393, 0.394, 0.395, 0.396, 0.397, 0.398, 0.399, or 0.4, the patient or sample is unclassified.

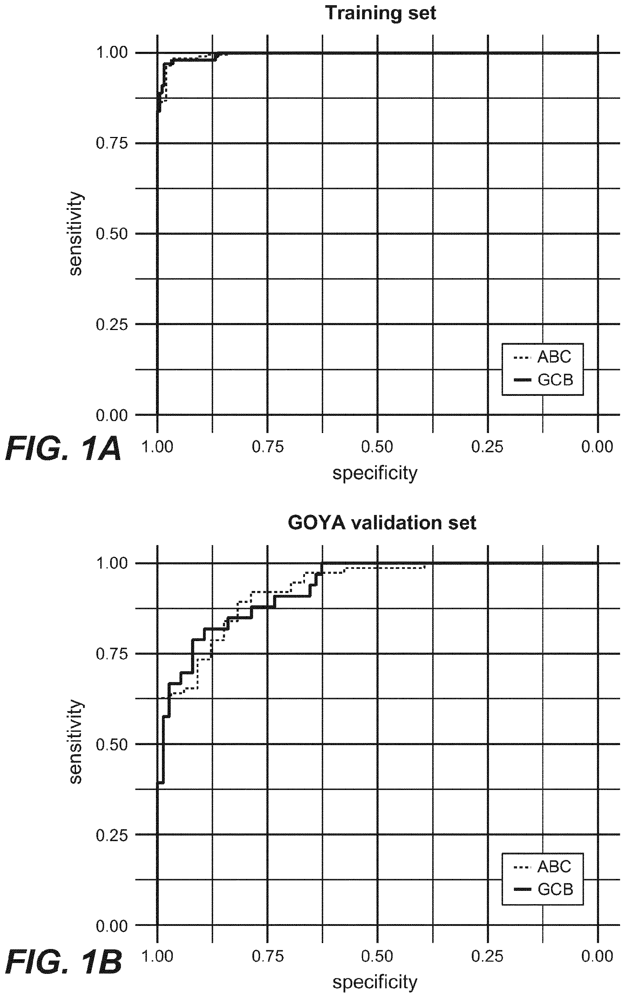

Without wishing to be bound by theory, it is believed that in some embodiments, the model described herein, which is described, e.g., by the features and weights, can be used to generate fitted predictor scores (e.g., probabilities) for the ABC subtype and these predictor scores (e.g., probabilities) can then be used to determine which subtype the patient or sample is based on pre-determined cutoffs. For example, the optimal cutoffs (e.g., the cutoffs described herein) for the continuous probability score can be determined by maximizing Youden's J statistic (the point with the highest total sensitivity and specificity) in the training set and then adding a 0.1 probability buffer on both sides of the initial cutoff to define an unclassified region, e.g., as described in Example 3 (e.g., Figures 1A and 1B). In certain embodiments, the cutoff, e.g., the low pre-defined cutoff described herein or the high pre-defined cutoff described herein, is determined as described in Example 3 (e.g., Figures 1A and 1B).

In some embodiments provided herein is a method for treating a subject having DLBCL comprising determining if the subject has an ABC or GCB COO, comprising i) acquiring, e.g., collecting, a sample, e.g., a clinical sample, and ii) performing a genotyping assay on the sample to determine if the subject has an ABC or GCB COO, and if the subject has DLBCL of ABC COO, then administering ibrutinib and/or lenalidomide, and if the subject has DLBCL of the GC COO, then administering ibrutinib and/or lenalidomide. In certain embodiments according to (or as applied to) any of the embodiments above, the COO is determined using a method or diagnostic of any of the embodiments described herein.

In some embodiments, provided herein is a computer system configured to perform any of the methods, diagnostics, kits or classifiers of any of the embodiments described herein.

### BRIEF DESCRIPTION OF THE DRAWINGS

**FIG. 1A** and **FIG. 1B** show receiver operating characteristics (ROC) curves for the COODC model. **FIG. 1A** shows ROC curves indicating sensitivity and specificity of the model for ABC and GCB for the GOYA training set and **FIG. 1B** shows ROC curves indicating sensitivity and specificity of the model for ABC and GCB for the GOYA held-out validation set.

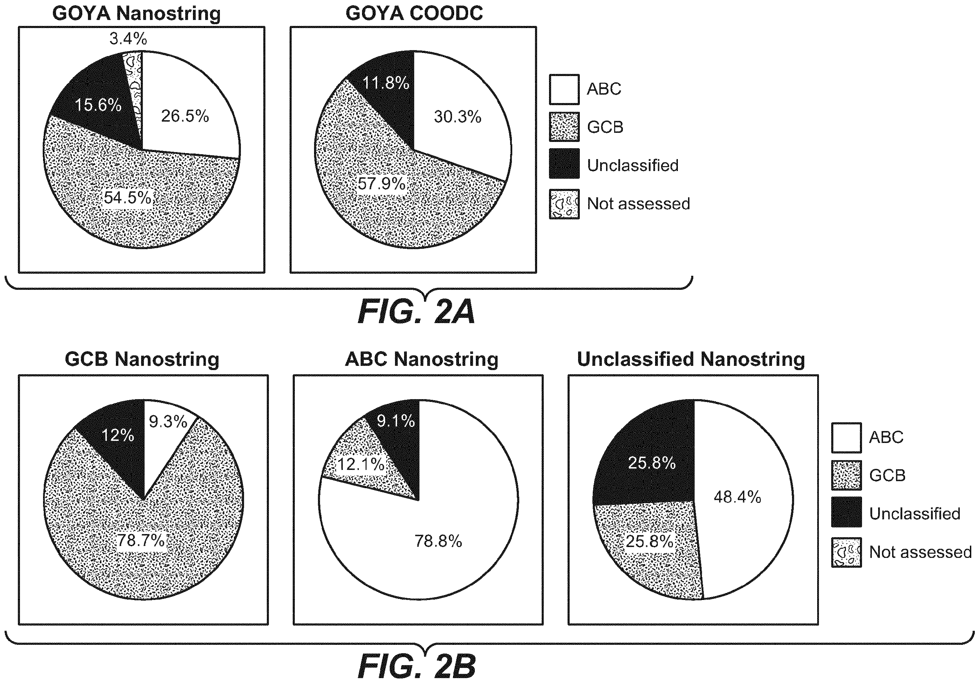

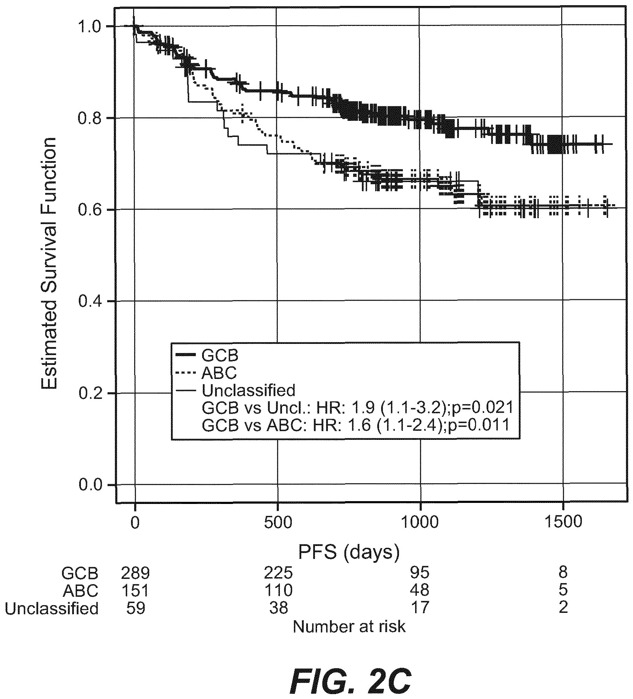

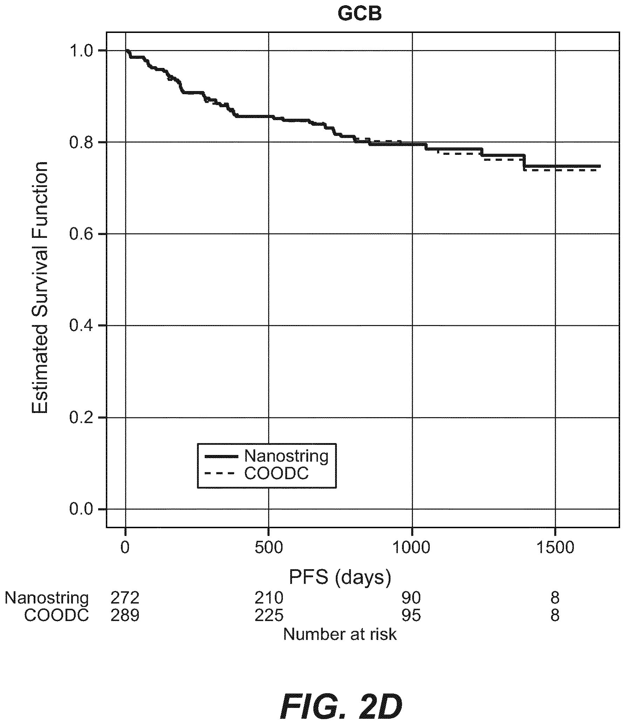

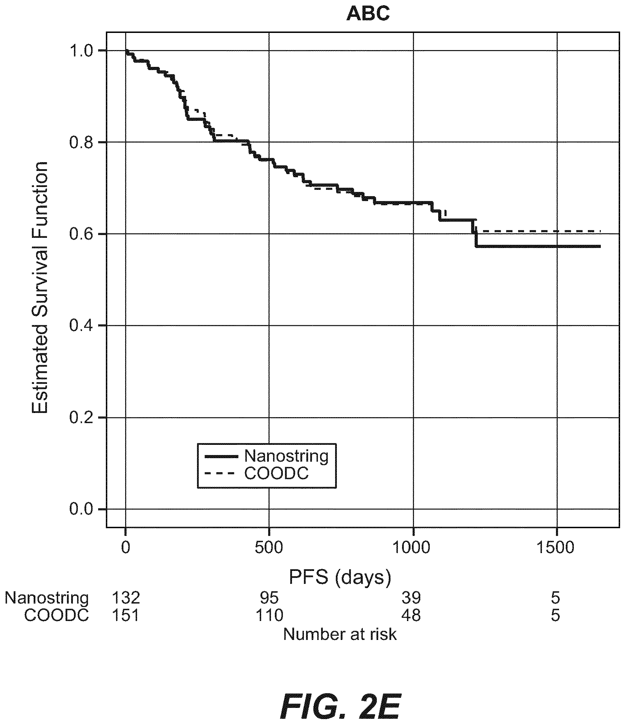

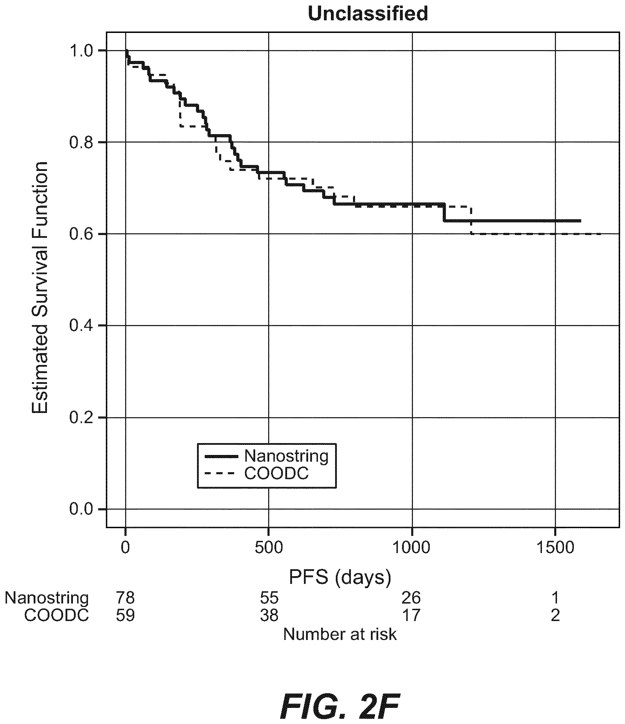

**FIGS. 2A****-2F** show concordance of COODC determined COO with Nanostring determined COO for GOYA samples. **FIG. 2A** shows pie graphs showing the overall breakdown of COO for all GOYA samples as determined by Nanostring or COODC. **FIG. 2B** shows pie graphs showing the breakdown of COODC COO calls within each Nanostring COO group. Survival curves indicating progression free survival for **(****FIG. 2C****)** COODC determined COO groups and **(****FIGs. 2D****,** **2E****,** **2F****)** COODC determined COO compared with Nanostring determined COO.

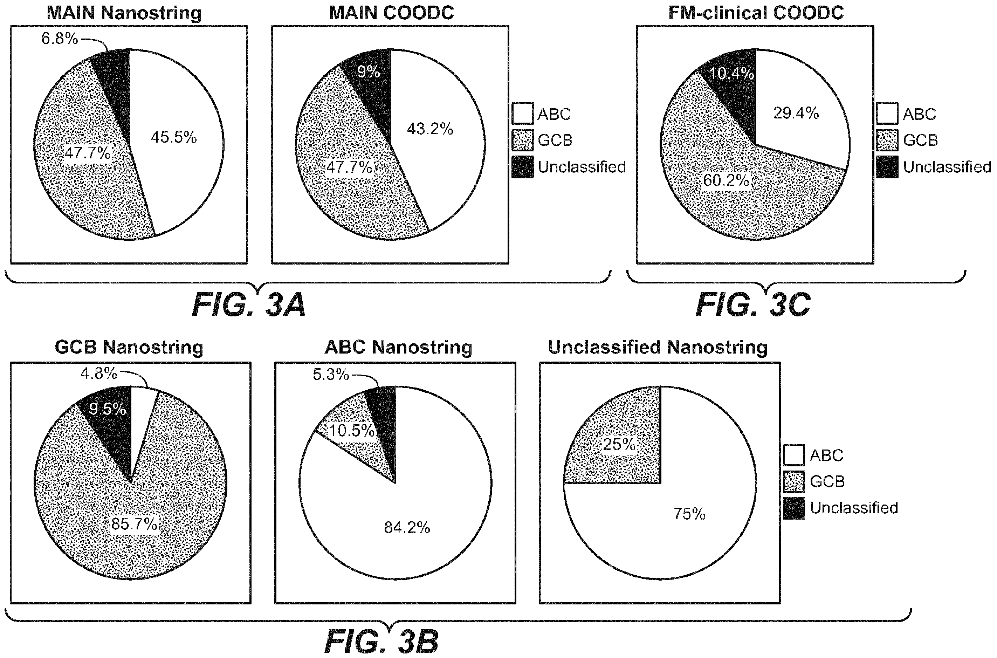

**FIGS. 3A****-3C** show concordance of COODC determined COO with Nanostring determined COO for MAIN samples. **FIG. 3A** shows pie graphs showing the overall breakdown of COO for all MAIN samples as determined by Nanostring or COODC. **FIG. 3B** **shows** pie graphs showing the breakdown of COODC COO calls within each Nanostring COO group for MAIN. **FIG. 3C** shows a pie graph showing overall breakdown of COO for Foundation Medicine's deidentified genomic research database ("FM-clinical") samples as determined by COODC.

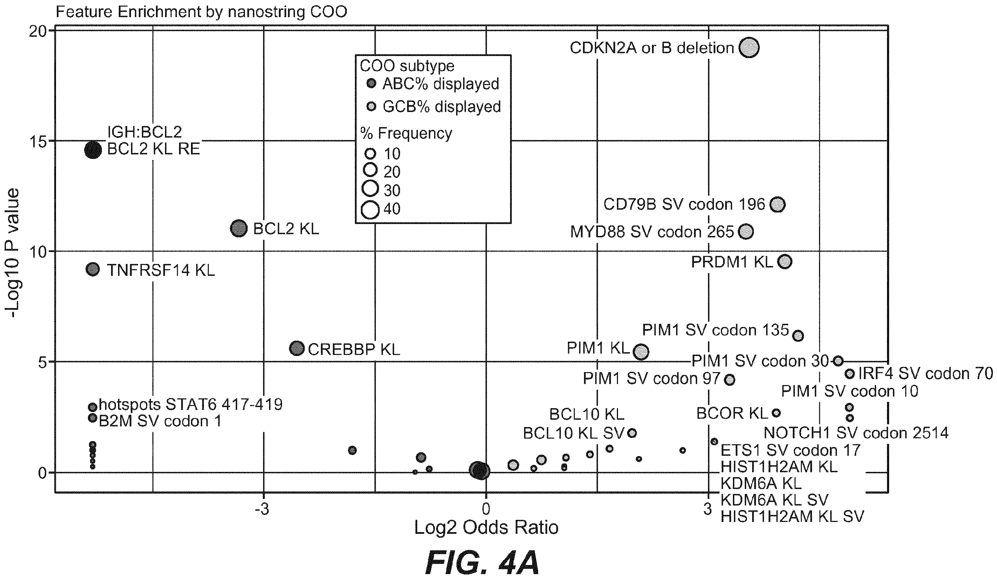

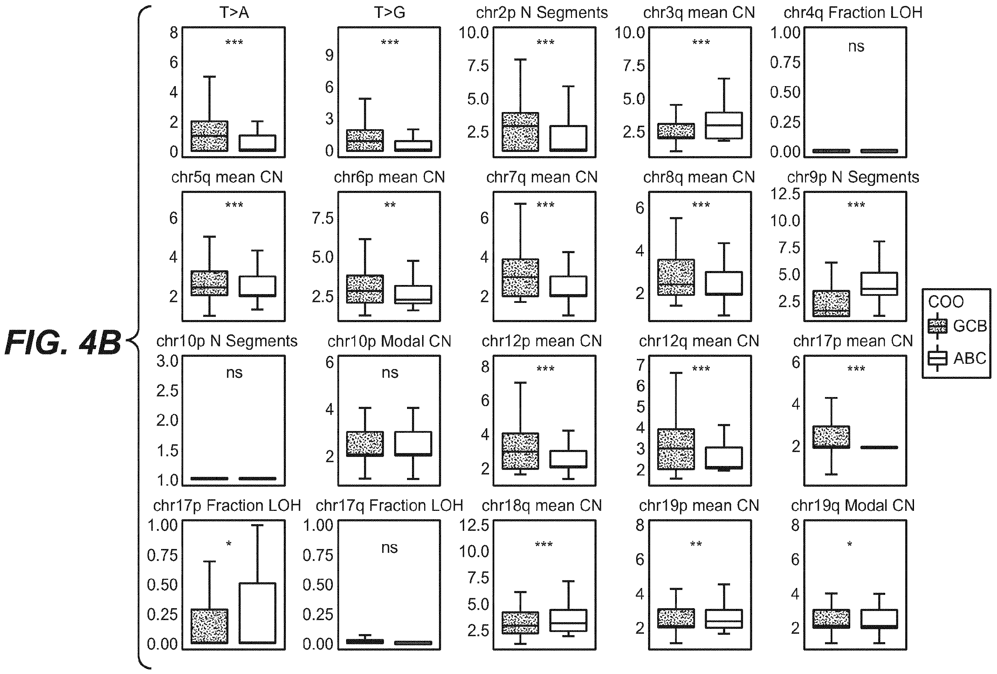

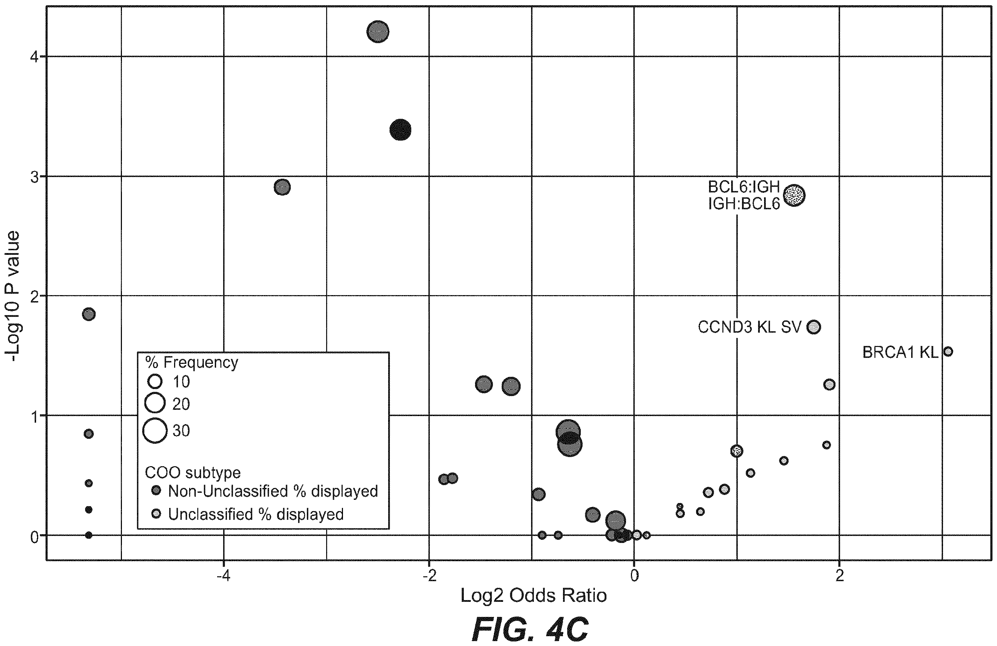

**FIG. 4A - 4C** show the enrichment of the COODC model features by COO. Enrichment is assessed using Nanostring assessed COO. **FIG. 4A** shows enrichment for all binary features with non-zero weights in the model. Enrichment in ABC is indicated by the darker grey dots (Log2 Odds Ratio >0), enrichment in GCB is indicated by orange dots (Log2 Odds Ratio <0). Dot sizes indicate the frequency of the feature in the enriched group. Labels indicate features with significant enrichment (p < 0.05). **FIG. 4B** shows enrichment for continuous features with non-zero weights in the model. GCB is always on the left hand side of each panel, and ABC is on the right hand side. The horizontal lines in each box represents the median of the feature while the lower and upper bounds of each box represent 25th percentile and 75th percentile, respectively. The whiskers associated with each box extend to extreme values of no more than 1.5 times the inter-quartile range beyond the applicable box. Points beyond whiskers are considered outliers and plotted individually. *** p < 0.001, ** p < 0.01, * p <0.05, ns=not significant. **FIG 4C** shows enrichment of COODC binary features in unclassified vs non-unclassified COO. Enrichment is assessed using Nanostring assessed COO. It shows enrichment for all binary features with non-zero weights in the model. Enrichment in unclassified is indicated by grey dots (Log2 Odds Ratio >0), and enrichment in non-unclassified is indicated by purple dots (Log2 Odds Ratio <0). Dot sizes indicate the frequency of the feature in the enriched groups. Labels indicate features with significant enrichment in unclassified samples (p < 0.05). Features enriched in non-unclassified are not shown, but rather ABC vs. GCB features are indicated in **FIG 4A****.**

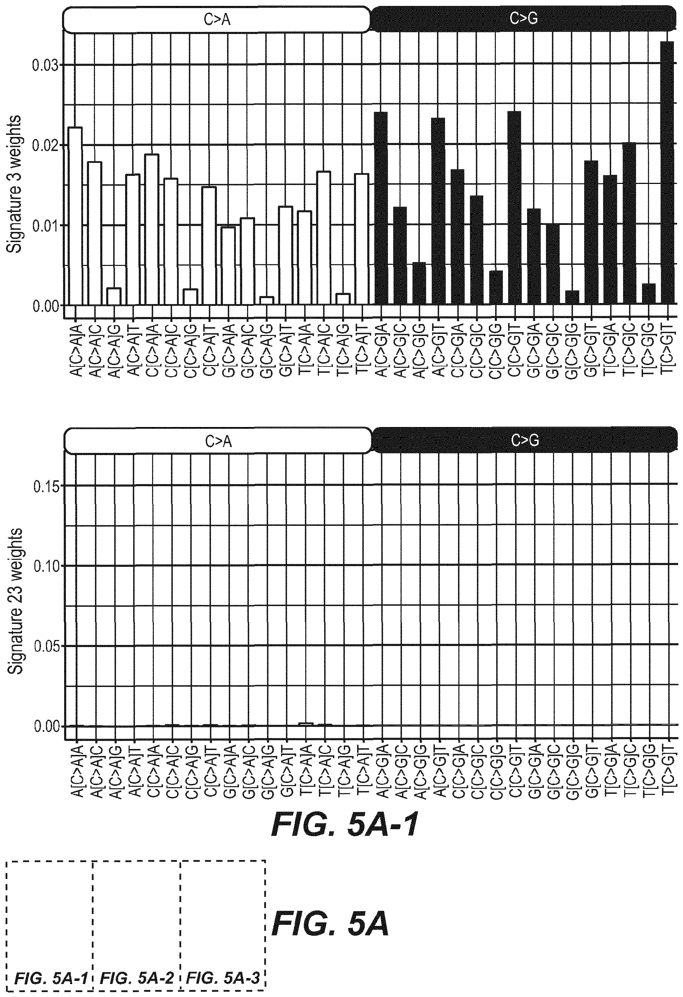

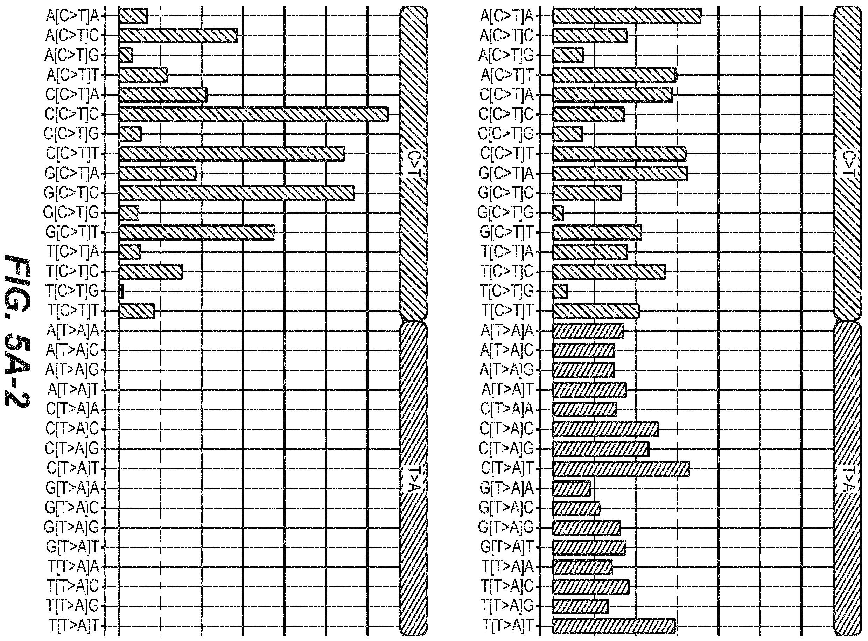

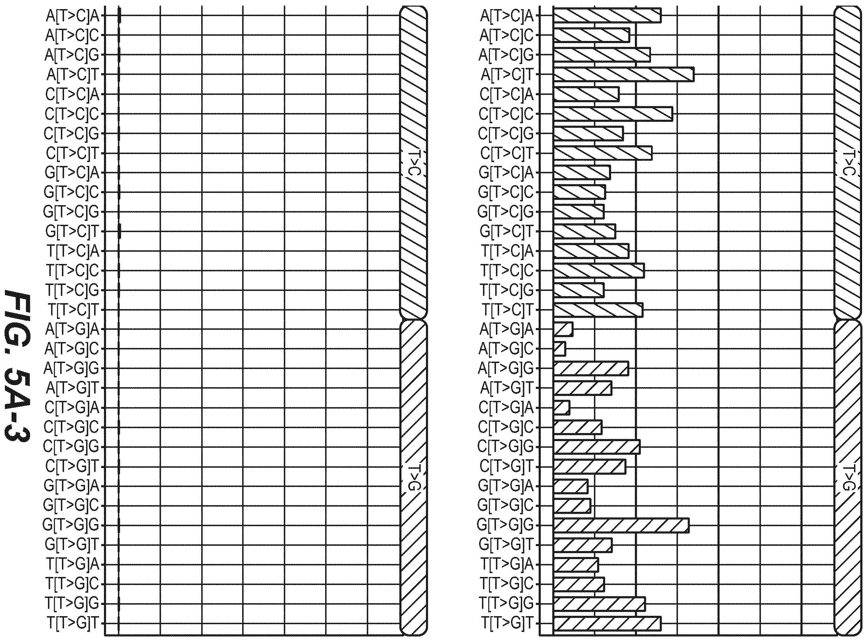

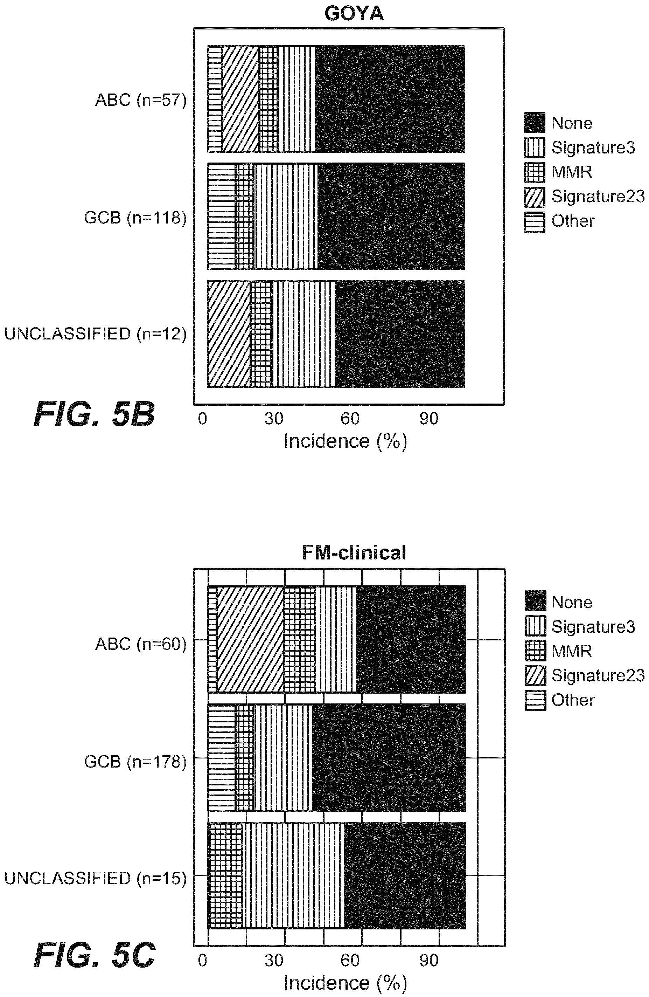

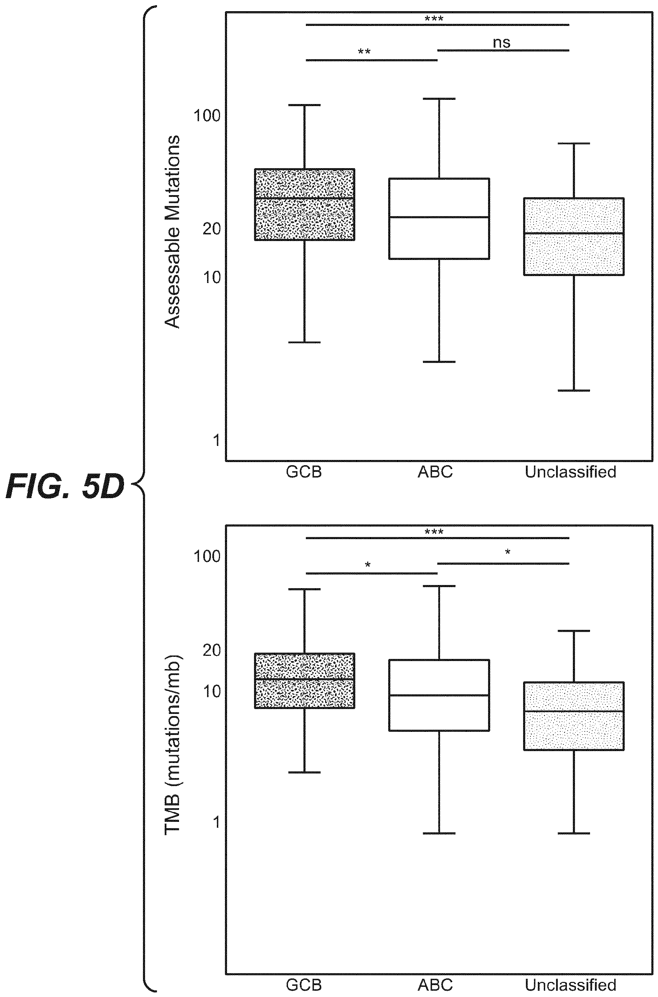

**FIGS. 5A****-5D** show mutation signatures by COO. **FIGS. 5A****-1-5A-3** shows plots of trinucleotide context for signature 3 ("BRCA" signature) (top) and signature 23 (bottom). **FIG. 5A** describes the positional arrangement between **FIGs. 5A****-1, 5A-2** and **5A-3.** **FIG. 5B** shows frequency of mutational signatures by COODC type for GOYA samples in baited genomic regions as described in the methods. **FIG. 5C** shows frequency of mutational signatures by COODC type for FM-Clinical samples in baited genomic regions as described in the methods. Only samples with >=20 assessable mutations are included. If no dominant signature (i.e., no signature with >0.4 score) was detected, the signature is reported as "None". **FIG. 5D** shows boxes (with whiskers) of all alterations assessed for trinucleotide context (left) and for TMB (right), with the median indicated by the horizontal line in each box. The lower and upper bounds of each box represent 25th percentile and 75th percentile, respectively. The whiskers associated with each box extend to extreme values of no more than 1.5 times the inter-quartile range beyond the box. Points beyond whiskers are considered outliers and plotted individually. *** p < 0.001, ** p < 0.01, * p <0.05, ns=not significant.

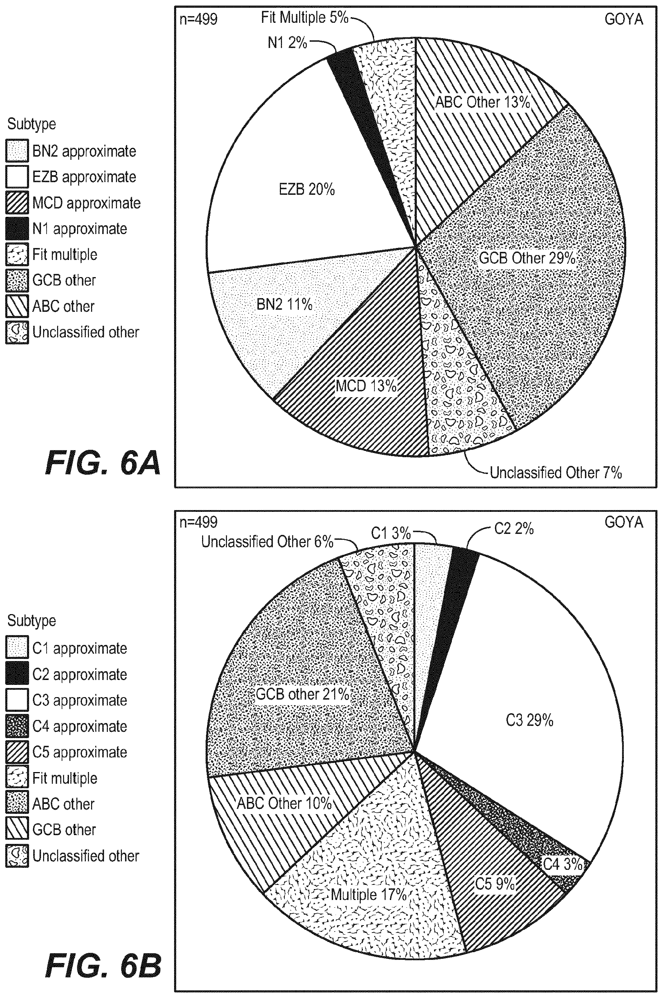

**FIG. 6A** and **FIG. 6B** show other genetically defined subsets of DLBCL in GOYA samples. **FIG. 6A** shows frequency of subsets approximating BN2, N1, MCD, and EZB groups identified by Schmitz and colleagues (Schmitz, R., et al. Genetics and Pathogenesis of Diffuse Large B Cell Lymphoma. New England Journal of Medicine 378, 1396-1407 (2018)). "Fit multiple" indicates samples that qualified for more than one genetically defined subset. Samples that were not qualified for any genetically defined subset were grouped based on the COODC subtype. **FIG. 6B** shows frequency of subsets approximating C1-C5 groups identified by Chapuy and colleagues (Chapuy, B., et al. Molecular subtypes of diffuse large B cell lymphoma are associated with distinct pathogenic mechanisms and outcomes. Nature Medicine 24, 679-690 (2018)). "Fit multiple" indicates samples that qualified for more than one genetically defined subset. Samples that were not qualified for any genetically defined subset were grouped based on the COODC subtype.

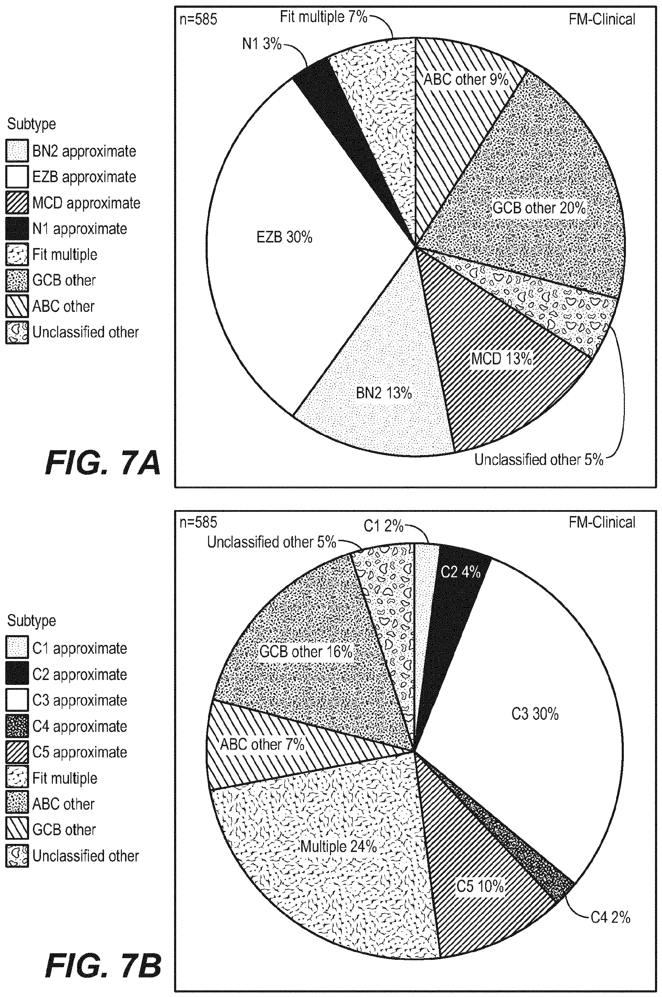

**FIG. 7A** and **FIG. 7B** show other genetically defined subsets of DLBCL in FM-clinical samples. **FIG. 7A** shows frequency of subsets approximating BN2, N1, MCD, and EZB groups identified by Schmitz and colleagues (Schmitz, R., et al. Genetics and Pathogenesis of Diffuse Large B Cell Lymphoma. New England Journal of Medicine 378, 1396-1407 (2018)). "Fit multiple" indicates samples that qualified for more than one genetically defined subset. Samples that were not qualified for any genetically defined subset were grouped based on the COODC subtype. **FIG. 7B** shows frequency of subsets approximating C1-C5 groups identified by Chapuy and colleagues (Chapuy, B., et al. Molecular subtypes of diffuse large B cell lymphoma are associated with distinct pathogenic mechanisms and outcomes. Nature Medicine 24, 679-690 (2018)). "Fit multiple" indicates samples that qualified for more than one genetically defined subset. Samples that were not qualified for any genetically defined subset were grouped based on the COODC subtype.

### DETAILED DESCRIPTION OF THE INVENTION

Disclosed herein is a new, clinically relevant, method for determining the COO for DLBCL samples with approximately 20% tumor purity (i.e., tumor content) and without the need for RNA or matched normal tissue. This method is ~90% concordant with the Nanostring assay compared to both a held-out validation set as well as an independent cohort. It was found that copy number alterations of chromosome 6p and 9p as well as the frequency of T>A and T>G transversions to be distinct between ABC and GCB subtypes. Finally, it was found that the mutational signatures underlying ABC are different from those found in GCB, suggesting differing roles of AID (activation-induced cytidine deaminase) in development of these two subtypes.

Current WHO guidelines require COO determination for newly diagnosed DLBCLs, but it is clinically challenging to obtain a standardized, rigorous determination for most samples (He, J., et al. Integrated genomic DNA/RNA profiling of hematologic malignancies in the clinical setting. Blood 127, 3004-3014 (2016)). IHC algorithms vary in both feasibility and utility of the results (Hans, C.P., et al. Confirmation of the molecular classification of diffuse large B Cell lymphoma by immunohistochemistry using a tissue microarray. Blood 103, 275-282 (2003); Gribben, R.C., et al. Poor Concordance among Nine Immunohistochemistry Classifiers of Cell-of-Origin for Diffuse Large B Cell Lymphoma: Implications for Therapeutic Strategies. (2013); Meyer, P.N., et al. Immunohistochemical Methods for Predicting Cell of Origin and Survival in Patients With Diffuse Large B Cell Lymphoma Treated With Rituximab. Journal of Clinical Oncology 29, 200-207 (2011)). The Nanostring assay has begun to emerge as a more clinically feasible alternative that evaluates the expression of a small subset of genes. However, the published validation for this assay, which is 98% concordant with the microarray assay, requires >60% tumor content as well as high quality RNA, which is often not available in the clinical setting (Scott, D.W., et al. Determining cell-of-origin subtypes of diffuse large B Cell lymphoma using gene expression in formalin-fixed paraffin-embedded tissue. Blood 123, 1214-1217 (2014)). Herein is described a new classifier that is ~90% concordant with Nanostring, requires only 20% tumor content (e.g., approximately 20% tumor content), and does not require RNA, which is more susceptible to degradation or lower quality than DNA. Based on data from FM-clinical samples, an estimated 50% of DLBCL samples would not be eligible or results may be unreliable for expression-panel COO determination such as Nanostring due to either missing RNA or having <60% tumor purity. Therefore, this method as applied clinically will greatly increase the number of samples with a reliable COO determination.

It has been shown that more granular classification of DLBCL utilizing genomic alterations can provide more insight into prognosis (Chapuy, B., et al. Molecular subtypes of diffuse large B cell lymphoma are associated with distinct pathogenic mechanisms and outcomes. Nature Medicine 24, 679-690 (2018); Schmitz, R., et al. Genetics and Pathogenesis of Diffuse Large B Cell Lymphoma. New England Journal of Medicine 378, 1396-1407 (2018)). The mutations utilized in the COO prediction model disclosed herein shared significant overlap with the mutations highlighted by these papers. As discovered and disclosed herein, the alterations in *BCL2, EZH2,* and *TNFRSF14* are all important predictors of the GCB subtype, consistent with the enrichment of GCB samples among the EZB cluster in Schmitz *et al.,* or the C3 group DLBCLs in Chapuy *et al.* (see references above). Similarly, mutations in *MYD88* and *CD79B* were highly predictive of an ABC subtype. Interestingly, it was discovered and disclosed herein that mutations in *NOTCH1 , NOTCH2,* and *BCL6* are all slightly predictive of ABC subtype, despite the mixed subtypes found in the BN2, N1 and C1 groups. However, these alterations were given less weight than other ABC-specific alterations, meaning that mutated samples would be more likely to fall into the "unclassified" subgroup.

It was also discovered and disclosed herein that many of the arm level copy number alterations in the COODC model are similar to the C2 group DLBCL in Chapuy et al. (see above), including 17p (both average copy number and fraction under loss of heterozygosity (LOH)), 6p (average copy number), and 5p (average copy number). In addition to these full arm level events, a number of partial arm level alterations correlate with full arm level alterations included in the COODC model such as 4q35.1 and 4q21.22 in the C2 group and 4q fraction under LOH in COODC. This suggests that many of the genomic groups identified by Schmitz and Chapuy overlap with the genomically defined COODC disclosed herein (see Schmitz *et al.,* and Chapuy *et al*)*.*

Approximate subsets similar to the BN2, N1, MCD, and EZB groups identified by Schmitz and C1-C5 groups identified by Chapuy and colleagues were used herein, illustrating that further subsetting into rough approximations of these populations should be feasible on a large scale clinical assay, which could provide more granular prognostic information. Additionally, provided herein are therapeutically relevant biomarkers, including BCR-dependence, *TP53* wild type, *EZH2* alterations, and *BCL2* alterations. Given the high frequency of samples in the DLBCL datasets used for the inventions and discoveries disclosed herein with biomarkers associated with targeted therapies (85% in GOYA study samples and 81% in FM-clinical samples), it is clear that adding COO calling capabilities to next generation sequencing (NGS) provides both prognostic and therapeutically relevant actionability information for DLBCL patients.

It was also found, using the DLBCL datasets disclosed herein, that there are similarities in mutational signatures to those identified by Chapuy.

Two major mutational signatures in DLBCL samples were discovered: COSMIC signature 3, annotated as BRCA signature, and COSMIC signature 23. COSMIC signature 23, which was found primarily in the ABC subtype, has frequent alterations in the R[C]Y context, which suggests a canonical AID signature. Interestingly, COSMIC signature 3 is common among all DLBCL subtypes, found in 26% of GCBs, 16% of ABCs, and 25% of unclassified from COODC GOYA samples when a signature could be determined. Given that COSMIC signature 3 appears to capture the effects of DSB resolved by non-homologous end joining (NHEJ) when BRCA1/2 is absent, this signature is likely capturing the resolution of AID-induced DSB, which also uses NHEJ (Kotnis, A., Du, L., Liu, C., Popov, S.W. & Pan-Hammarström, Q. Non-homologous end joining in class switch recombination: the beginning of the end. in Philos Trans R Soc Lond B Biol Sci, Vol. 364 653-665 (2009)), and is essential for CSR.

Additionally, it was discovered that almost exclusively COSMIC signature 3 is found across all subtypes region of the IGH locus, suggesting a shared mutational process.

These findings fit in a model whereby GCBs originate from a cell that has experienced the mutational stress of AID mediated DSB, while ABCs originate from a cell that has undergone mutational stress related to both AID-mediated DSB and AID-mediated single base alterations. This fits with the current model in which GCBs originate from an earlier germinal center B Cell, while ABCs originate from a developmentally more advanced post or late germinal center cell. This is especially interesting given that there is only a small difference in tumor mutational burden (TMB) between ABC and GCB subtypes (median ABC 8.9, GCB 12.1, p=0.011), and that there is a slightly lower number of mutations assessable for mutational signature calling (including silent and non-coding alterations) in ABC than in GCB (median ABC 23.5, GCB 31, p=0.0019). This suggests that, while some ABC cells experience both AID-mediated DSB and AID-mediated single base alterations, and while GCB cells appear to mainly experience AID-mediated DSB, the mutational processes that lead to transformation result in similar overall mutational burden.

In certain embodiments according to (or as applied to) any of the embodiments above, "sample" refers to a biological sample obtained or derived from a source of interest, as described herein. In some embodiments, a source of interest comprises an organism, such as an animal or human. The source of the sample can be solid tissue as from a fresh, frozen and/or preserved organ, tissue sample, biopsy, resection, smear, or aspirate; blood or any blood constituents; bodily fluids such as cerebral spinal fluid, amniotic fluid, peritoneal fluid or interstitial fluid; or cells from any time in gestation or development of the subject. In some embodiments, the source of the sample is blood or blood constituents.

In some embodiments, the sample is or comprises biological tissue or fluid. The sample can contain compounds that are not naturally intermixed with the tissue in nature such as preservatives, anticoagulants, buffers, fixatives, nutrients, antibiotics or the like. In one embodiment, the sample is preserved as a frozen sample or as formaldehyde- or paraformaldehyde-fixed paraffin-embedded (FFPE) tissue preparation. For example, the sample can be embedded in a matrix, e.g., an FFPE block or a frozen sample. In another embodiment, the sample is a blood or blood constituent sample. In yet another embodiment, the sample is a bone marrow aspirate sample. In another embodiment, the sample comprises cell-free DNA (cfDNA). Without wishing to be bound by theory, it is believed that in some embodiments, cfDNA is DNA from apoptosed or necrotic cells. Typically, cfDNA is bound by protein (*e.g.,* histone) and protected by nucleases. CfDNA can be used as a biomarker for non-invasive prenatal testing (NIPT), organ transplant, cardiomyopathy, microbiome, and cancer. In another embodiment, the sample comprises circulating tumor DNA (ctDNA). Without wishing to be bound by theory, it is believed that in some embodiments, ctDNA is cfDNA with a genetic or epigenetic alteration (e.g., a somatic alteration or a methylation signature) that can discriminate it originating from a tumor cell versus a non-tumor cell. In another embodiment, the sample comprises circulating tumor cells (CTCs). Without wishing to be bound by theory, it is believed that in some embodiments, CTCs are cells shed from a primary or metastatic tumor into the circulation. In some embodiments, CTC apoptosis is a source of ctDNA in the blood/lymph.

In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or bronchoalveolar lavages; aspirates; scrapings; bone marrow specimens; tissue biopsy specimens; surgical specimens; feces, other body fluids, secretions, and/or excretions; and/or cells therefrom, etc.

In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by a method chosen from biopsy (e.g., fine needle aspiration or tissue biopsy), surgery, collection of body fluid (e.g., blood, lymph, or feces), etc. In some embodiments, as will be clear from context, the term "sample" refers to a preparation that is obtained by processing (e.g., by removing one or more components of and/or by adding one or more agents to) a primary sample, e.g., filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

In some embodiments, the sample is a cell associated with a tumor, *e.g.,* a tumor cell or a tumor-infiltrating lymphocyte (TIL). In some embodiments, the sample includes one or more premalignant or malignant cells. In some embodiments, the sample is acquired from a hematologic malignancy (or premaligancy), *e.g.,* a hematologic malignancy (or premaligancy) described herein, *e.g., a* diffuse large B-cell lymphoma (DLBCL). In other embodiments, the sample includes one or more circulating tumor cells (CTCs) *(e.g.,* a CTC acquired from a blood sample). In some embodiments, the sample is a cell not associated with a tumor, *e.g.,* a non-tumor cell or a peripheral blood lymphocyte.

### EXAMPLES

### Example 1: Samples

Clinical and genomic data was available from patients treated in the GOYA clinical trial (NCT01287741; R-CHOP vs G-CHOP) (Vitolo, U., et al. Obinutuzumab or Rituximab Plus Cyclophosphamide, Doxorubicin, Vincristine, and Prednisone in Previously Untreated Diffuse Large B Cell Lymphoma. Journal of Clinical Oncology 35, 3529-3537 (2017)) and MAIN clinical trial (NCT00486759; R-CHOP +/- Bevacizumab) (Seymour, J.F., et al. R-CHOP with or without bevacizumab in patients with previously untreated diffuse large B Cell lymphoma: final MAIN study outcomes. Haematologica 99, 1343-1349 (2014)). The protocols of the original trials were approved by local or national ethics committees according to the laws of each country, and the studies were undertaken in accordance with the Declaration of Helsinki. Activated B Cell (ABC)/germinal center B Cell (GCB)/Unclassified DLBCL prognostic subtypes were determined by the Nanostring assay. Median follow-up durations were 24 months and 29 months for MAIN and GOYA, respectively (≈ 30% of progression free survival (PFS) events in both cohorts). Routine clinical care samples (hereafter referred to as FM-clinical samples) were sequenced as part of routine clinical care and corresponding clinical data was not available.

### Example 2: DNA Sequencing

All samples were sequenced using the DNA component of the FoundationOne^{®}Heme platform, which includes targeted DNA-sequencing of approximately 465 genes, as described in He, J., et al. Integrated genomic DNA/RNA profiling of hematologic malignancies in the clinical setting. Blood 127, 3004-3014 (2016). In addition to validated short variants, copy number alterations (high-level amplifications and deep deletions), rearrangements, tumor mutational burden (TMB), and microsatellite instability (MSI) status, research use only features of the platform were utilized including chromosomal arm level copy number and loss of heterozygosity (LOH) metrics.

### Example 3: Machine Learning

The COODC model was developed using a penalized Lasso regression using 25-fold internal cross validation implemented from the glmnet package (version 2.0-10) in R version 3.3.2 and using RStudio version 1.0.136. 482 GOYA samples with Nanostring data were split into a training set (70% of the samples) and a validation set (30% of the samples). The initial training set was further refined by removing Nanostring unclassified samples to focus the training to make ABC or GCB calls. 296 samples were used for the final training set, while 139 samples were used for the validation set. 592 features were used in the model. Continuous features were z-scored to maintain a consistent scale between continuous and binary features. The final COODC model included 74 non-zero features **(Table 1).** Per sample probabilities extracted from the model were used to determine the ideal cutoffs. ROC curves were generated **(****Figures 1A and 1B****)** and the "best" cutoff, which optimizes specificity and sensitivity, was chosen. 10% was then added on either side of the optimal cutoff to generate an unclassified zone. The probabilities were then extracted for the held-out validation set and 44 independent validation samples from the MAIN study. These two validation sets were used to determine the accuracy of the model.

**Table 1: Features of the COODC model**

| Features | Coefs |
|---|---|
| (Intercept) | 0,74622792 |
| T.A | 0.1466164 |
| T.G | 0.19169638 |
| chr2p Num Segments | 0.12336813 |
| chr3q Average CN | -0.5625825 |
| chr4q Fraction LOH | 0,00273763 |
| chr5q Averge CN | 0.00758733 |
| chr6p Average CN | 0.00369147 |
| chr7q AVerage CN | 0.22046432 |
| chr8q Average CN | 0.00869661 |
| chr9p Num Segments | -0.0870849 |
| chr10p Num Segments | -0.0132862 |
| chr10p Modal CN | 0.04487006 |
| chr12p Average CN | 0.10812066 |
| chr12q Average CN | 0.33833335 |
| chr17p Average CN | 0.06825782 |
| chr17p Fraction LOH | -0.0668038 |
| chr17q Fraction LOH | 0.0772327 |
| chr18q Average CN | -0.0135074 |
| chr19q Average CN | -0.2309455 |
| chr19q Modal CN | -0.0159153 |
| ft : PIM1 \| isKL - equals - KL | -0.2789378 |
| ft : NOTCH1 \| variant type - equals - SV + codon - equals - 2514 | -0.7118127 |
| ft : BCOR \| variant type - equals -SV + isKL - equals - KL | -0.4570838 |
| ft : TNFRSF14 \| isKL - equals - KL | 0.30795551 |
| ft : BCL2 \| isKL - equals - KT | 0.91799866 |
| ft : HIST1H2AM \| isKT - equals - KL | -1.1452282 |
| ft : CREBBP \| isKL - equals - KL | 0.39528297 |
| ft : GNA13 \| variant type - equals - SV + codon - equals - 28 | 0.04776333 |
| ft : BCL10 \| variant type - equals - SV + isKL - equals - KL | -0.657969 |
| ft : KMT2D \| variant type - equals - SV + isKL - equals - KL | -0.0542613 |
| ft : BRCA1 \| isKTL - equals - KL | -0.2540207 |
| ft : DUSP2 \| variant type - equals - SV + codon - equals - 134 | -0.8152089 |
| ft : PIM1 \| variant type - equals - SV + codon - equals - 135 | -0.1497665 |
| ft : PTPN11 \| isKTL - equals - KL | -0.1167284 |
| ft **:** BTG2 \| variant type - equals - SV + codon - equals - 48 | -0.0152634 |
| ft : BTG2 \| variant type - equals - SV + codon - equals - 45 | 0_{.}02565534 |
| ft : CCND3 \| variant type - equals - SV + codon - equals - 190 | 0.57357736 |
| ft **:** DAXX \| variant type - equals - SV + codon - equals - 455 | -0.3863176 |
| ft : FBXO11 \| isKL - equals - KL | 0.12557906 |
| ft : MAPK1 \| variant type - equals - SV + codon - equals - 1 | 0.020638698 |
| ft : IGH \| variant type - equals - RF + gene2 - equals - BCL2 + isKL - | 0.42225842 |
| ft : KDM6A \| variant type - equals - SV + isKL- equals - KL | -0.382399 |
| hotspots STAT6 417 419 | 0.42139716 |
| ft : BCL6 \| variant type - equals - RF + gene2 - equals - IGH + isKL - | -0.1259457 |
| ft : B2M \| variant type - equals - SV + codon - equals - 1 | 0.74380662 |
| ft : PTPN6 \| variant type - equals - SV + isKL - equals - KL | -0.1007704 |
| ft : TP53 \| variant type - equals - SV + codon - equals - 273 | 0.00758395 |
| ft **:** KMT2D \| isKTL -equals - KL | -0.0167986 |
| ft : HTST1H1E \| variant type - equals - SV + isKL - equals - KL | 0.18457867 |
| ft : CARD11 \| variant type - equals - SV + codon - equals - 230 | -0.0008312 |
| CDKN2A or B del | -0.6592701 |
| ft : PTPN11 \| variant type - equals - SV + codon - equals - 197 | -0.0001514 |
| ft : PIM1 \| variant type - equals - SV + codon - equals - 97 | -0.8007032 |
| ft : CD79B \| variant type - equals - SV + codon - equals - 196 | -0.0960342 |
| ft : CDKN2B \| variant type - equals - RF + isKL - equals - KL | -0.7328949 |
| ft : MYC \| variant type - equals - RF + gene2 - equals - IGH + isKL - equals - | -0.0427746 |
| ft : MYD88 \| variant type - equals - SV + codon - equals - 265 | -0.359751 |
| ft : DDX3X \| variant type - equals - SV + isKL - equals - KL | 0.06998015 |
| ft : PIM1 \| variant type - equals - SV + codon - equals - 30 | -0.0375632 |
| ft : BCL10 \| isKTL - equals - KL | -0.1240489 |
| ft : TBL1XR1 \| variant type - equals - SV + isKL - equals - KL | -0.4020574 |
| hotspots KLHL6 547 568 65 90 | 0.0732899 |
| ft : IGH \| variant type - equals - RF + gene2 - equals - BCL6 + isKL - | -0.0026765 |
| ft : IRF4 \| variant type - equals - SV + codon - equals - 70 | -0.2206269 |
| ft : PRDM1 \| isKL - equals - KL | -0.3567579 |
| ft : COND3 \| variant type - equals - SV + isKL - equals- KL | -0.0157609 |
| ft : BCL2 \| variant type - equals - RF + isKL - equals - KL | 0.02093477 |
| ft : HTST1H2AM \| variant type - equals - SV + isKL - equals - KL | -0.0517756 |
| ft : KLHL6 \| variant type - equals - SV + codon - equals -568 | 0.03590108 |
| ft : KDM6A \| isKTL - equals - KL | -0.0095082 |
| ft : PTPN6 \| isKL - equals - KL | -0.0194835 |
| ft : PTPN11 \| variant type - equals - SV + isKL - equals - KL | -0.0014204 |
| ft : ETS1 \| variant type - equals - SV + codon - equals - 17 | -0.2238722 |
| ft : PIM1 \| variant type - equals - SV + codon - equals - 10 | -0.0111813 |

### Example 4: Statistics

Feature enrichment was assessed using the Nanostring assigned COO. Binary feature enrichment was determined using a Fisher exact test. Continuous feature enrichment was determined using a Mann-Whitney-Wilcox test. Univariate Hazard ratios and p-values for the association of COO subtypes with PFS were calculated using Cox regression.

### Example 5: Mutational Signatures

Mutational signatures were determined as described by Zehir et al. (Zehir, A., et al. Mutational landscape of metastatic cancer revealed from prospective clinical sequencing of 10,000 patients. Nat Med 23, 703-713 (2017)). Briefly, trinucleotide matrices were decomposed into the 30 COSMIC signatures (Alexandrov, L.B., et al. Signatures of mutational processes in human cancer. Nature 500, 415-421 (2013)). Signatures were aggregated to APOBEC (signatures 2 and 13); smoking (signature 4), BRCA (signature 3); MMR (signatures 1, 6, 15, 20 and 26); UV (signature 7); POLE (signature 10); and Alkylating (signature 11). All point mutations were included in the analysis with the exception of driver alterations and predicted germline alterations. A sample was deemed to have a dominant signature if a mutational class harbored a score of 0.4 or greater. For the genome-wide calculation, mutations were considered in all regions baited for short variant detection excluding regions with known sequencing artifacts. Samples were only considered if they had 20 or more assessable mutations. For the IGH analysis, mutations were assessed in selected regions of the genomic IGH locus (chr14:106211312-106382700 hg19).

### Example 6: COO DNA Classifier: a genomically defined model for cell of origin

482 DLBCL samples from the GOYA study that had DNA sequenced using FoundationOne^{®}Heme were split into two sets: 2/3 into a training set and 1/3 into a held-out validation set. The training set was further subset to include only samples with either an ABC or GCB call as determined by Nanostring; unclassified samples or samples without a call were excluded. The held-out validation set was used after the model was complete to determine concordance. The training set data were then used to train a penalized logistic regression model to identify ABC or GCB samples using DNA-based features without the need for RNA. 594 features were available to train the model, including binary features of any alteration in a gene, specific alterations (codons), and hotspot alterations (any one of multiple codons) that occurred at least 5 times in the GOYA dataset as well as derived DNA-based features such as tumor mutational burden (TMB), chromosome arm-level copy number and zygosity metrics, and frequency of alteration classes (e.g., T mutated to A). Per-sample probabilities were extracted from the model, and a pair of cutoffs was chosen to optimize sensitivity and specificity, with particular focus on optimizing ABC accuracy. This model is herein referred to as the Cell of Origin DNA Classifier (COODC; the classifier is also referred to herein, e.g., as a method, model and assay). The COODC model contained a total of 74 genomic features and generated a continuous probability score of a sample being ABC ranging from 0 to 0.999. The 74 genomic features included 18 arm-level alteration features, including copy number and loss of heterozygosity features, 32 gene short variant features, 6 rearrangement-based features, 13 gene-level features (including copy number, rearrangement, and short variant alterations) and various other summary features (including T>A mutation prevalence).

To define optimal cutoffs for the continuous probability score, the "best" cutoff from the ROC model **(****Figures 1A** and **1B****)** was determined, i.e. the point with the highest total sensitivity and specificity. A 0.1 probability buffer was then added on either side of the optimal cut-point in order to define an unclassified region. This resulted in similar subtype breakdowns across the entire GOYA dataset, with 57.9% GCB, 30.3% ABC, and 11.8% unclassified as determined by the COODC compared to 54.5% GCB, 26.5% ABC, and 15.6% unclassified (3.4% of cases were not submitted for Nanostring COO typing), as determined by Nanostring **(****Figure 2A****).** This results in 85 (88.5%) concordant calls within the held-out validation set when considering 96 samples with either an ABC or GCB call from both the COODC and Nanostring methods (unclassified samples by either method were excluded) **(Table** 2). For this assessment, all unclassified calls were excluded. However, in keeping with the assessment provided by Scott et al. (Scott, D.W., et al. Determining cell-of-origin subtypes of diffuse large B Cell lymphoma using gene expression in formalin-fixed paraffin-embedded tissue. Blood 123, 1214-1217

(2014)), where only unclassified from the 'gold standard' were excluded, it was found 90% (97/108) concordance with Nanostring **(Table 2).** Of 75 samples Nanostring considered GCB, 59 (78.7%) were also called GCB by COODC, 9 (12%) were called unclassified, and 7 (9.3%) were incorrectly called ABC **(****Figure 2B** and **Table 2).** Similarly, of 33 ABC calls by Nanostring, 78.8% (26) were called ABC by COODC, 9.1% (3) were called unclassified, and 12.1% (4) were incorrectly called GCB **(****Figure 2B** and **Table 2).** In addition, the concordance with Nanostring was assessed on an independent study cohort (MAIN study) of 44 samples. In this cohort, COODC demonstrated continued high concordance with 91.9% accuracy **(****Figures 3A, 3B,** and **3C** and **Table 3).** The COODC model was further applied to an independent set of 597 FM-clinical samples **(****Figures 3A, 3B,** and **3C****).** Although there is no gold standard COO assessment to compare with, similar breakdowns of COO type by COODC were found as in the GOYA samples, with 60% GCB, 30% ABC, and 10% unclassified.

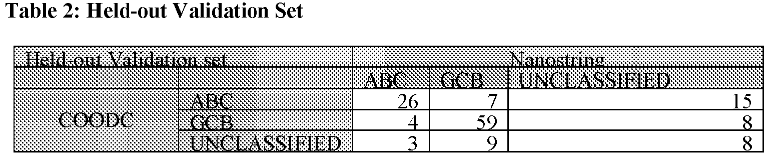

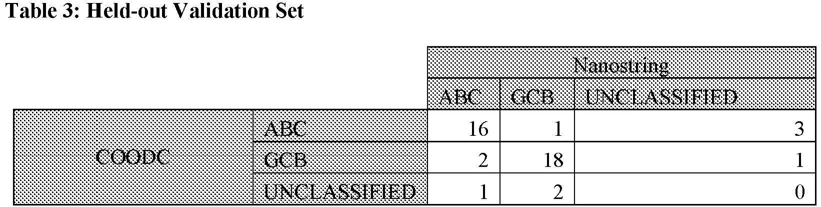

Overall, progression free survival (PFS) estimates for the COODC classifications were highly consistent with the Nanostring COO calls **(****Figure** 2D), and it was observed to significantly reduced PFS among samples classified as ABC (HR: 1.6; 95% confidence interval (CI) [1.1-2.4]; p=0.011) or unclassified (HR: 1.9; 95% CI [1.1-3.2]; p=0.021) when compared to GCB samples. This indicates that stratifying patient prognosis can be achieved utilizing a DNA only COO model such as COODC.

### Example 7: Biological significance of features included in the COODC model

Because an unbiased approach was taken to feature selection, the potential biological significance of the features included in the COODC model was of interest. It was found that a number of expected features, including *IGH:BCL2* rearrangements, *CREBBP* alterations, and *TNFRSF14* alterations are all highly enriched in GCB samples. Chromosome 3q copy number increase, *CDKN2A*/*B* deletions, *CD79B* alterations at amino acid 196, and *MYD88* alterations at amino acid 265 were highly enriched in ABC samples **(****Figure 4A****).** A few new features of particular interest, including the frequency of T>A and T>G alterations, the average copy number of chromosome 6p and the number of copy number segments of chromosome 9p **(****Figure 4B****)** were also identified. Both T>A and T>G alteration frequencies in non-driver alterations is higher in GCB (p<0.0001 for both) **(****Figure 4B****).** A lower mean copy number of chromosome 6p, which encodes the HLA locus, was found in ABC (p=0.0064). The number of modeled segments on chromosome 9p, including *CD274* and *PDCD1LG2,* is higher in ABC (p<0.0001) suggesting that regions of this arm are fractured, leading to differences in copy number. These two components, which include the number of modeled segments on chromosome 9p and the lower copy number of chromosome 6p, point to potential biological significance for immune evasion.

In addition to features specifically enriched in ABC or GCB, there was evidence of the truly intermediate nature of unclassified samples. Enrichment of novel features was identified, such as *IGH:BCL6* rearrangements (Figure 4C), as well as enrichment of ABC or GCB-like features, such as the average copy number of chromosome 6p in which unclassified is similar to GCB, and the T>A alteration frequency in which unclassified is similar to ABC (Figure 4B). Many of the features included in the model were not significantly enriched in ABC or GCB subtypes. This suggests that some features may be important aspects of prediction only in combination with or in the absence of other features.

### Example 8: Mutational Signatures

The observed difference in T>A and T>G alterations resulted in an investigation of potential differences in mutational signatures. Given the known role of AID in SHM and CSR in normal germinal center B Cells, the proposed role in transformation to lymphoma, and the recent data from Chapuy showing distinct mutational signatures in DLBCL, it was investigated whether there was evidence of the AID mutational processes across all the DNA alterations. Using trinucleotide mutational signatures identified in COSMIC, it was found that 26.3% of COODC-GCB samples compared to 15.8% of ABC samples (p =0.13) have a dominant COSMIC signature 3, while 14% of ABC samples compared to 1.7% of GCB samples (p = 0.002) have a dominant signature 23 **(****Figure 5B****).**

Signature 23 is dominated by the trinucleotide context in which C is altered to T **(****Figure 5A****).** Of the main trinucleotide contexts, 3/5 are found in the R[C]Y context **(****Figure 5A****).** This signature is similar to WR[C]Y, a common context for AID targeting somatic hypermutation, suggesting a strong AID mutational signature in ABC. Notably, this was not the result of fewer mutations overall in the GCB subset; the total mutation count was higher on average in GCB subsets compared with ABC (31 in GCB vs 23.5 in ABC, p=0.002) **(****Figure 5D**). Interestingly, the predominant COSMIC signature in GCB samples is signature 3, which has been annotated as a "BRCA" signature. Given the similarities between resolution of DSB in the absence of BRCA, which utilizes non-homologous end joining (NHEJ) and the resolution of DSB induced by AID as part of CSR, which also primarily utilizes NHEJ, it is presumed that signature 3 may identify the mutational scars of DSB repair, and in this context, may represent an AID-DSB signature.

### Example 9: Other genetically-defined subsets of DLBCL

In light of the recent publications highlighting the use of genetically defined DLBCL subsets to stratify further good and poor prognosis groups, subsets in data from this targeted NGS panel were investigated. Given the similarities between the subsets identified by Schmitz *et al.* and Chapuy *et al.* (MCD is similar to C5, EZB is similar to C3, and BN2 is similar to C1 as classified by Schmitz and Chapuy respectively), approximate definitions corresponding to the "seed" for these specific subsets, including CD79B alterations or MYD88 L265P for MCD/C5, BCL6 rearrangement or NOTCH2 alteration for BN2/C1, EZH2 alteration or BCL2 rearrangement for EZB/C3, and NOTCH1 alteration for N1 were identified.

Using these simplified genetically defined groups, 46% of GOYA and 57% of FM-clinical samples could be classified into one of these groups, while 49% of GOYA and 37% of FM-clinical samples could not be, and the remaining 5% of GOYA and 6% of FM-clinical samples could be classified into more than one of these genetically defined groups **(****Figures 6A** and **6B****,** **Figures 7A** and **7B****).**

In addition to these groups that are similar to those recently defined as potentially of prognostic value by Schmitz and colleagues and Chapuy and colleagues, subgroups with therapeutic value **(Table 4** and **Table 5)** were identified using the COODC disclosed herein. It was possible to identify BCR-independent and BCR-dependent subgroups using *CD79B* alterations with ABC, with predictive implications for ibrutinib as shown by Wilson and colleagues (Wilson, W.H., et al. Targeting B cell receptor signaling with ibrutinib in diffuse large B cell lymphoma. Nature Medicine 21, 922 (2015)). It was also found that a BCR-dependent subgroup, defined by the presence of a *CD79B* predicted deleterious alteration, are present in 24.5% of the ABC GOYA study samples (9.4% overall in GOYA) and 21.9% of the ABC FM-clinical samples (7.9% overall in FM-clinical samples). It was additionally observed that 79.2% of GOYA samples and 63.8% of FM-clinical samples were *TP53* wild type, suggesting a potential relevance for MDM2 inhibitors in these cohorts. EZH2 inhibitors may be clinically relevant in the 10% of GOYA samples and 12.7% of FM-clinical samples with EZH2 alterations, while BCL2 inhibitors could be considered in the 23.2% of GOYA samples and 32.5% of FM-clinical samples with *IGH:BCL2* rearrangements, *BCL2* amplifications, or *BCL2* short variants **(Table 4** and **Table 5).** Overall, this suggests that up to 85% of the DLBCL samples in the tested cohorts could be eligible for targeted therapy using an MDM2 inhibitor, an EZH2 inhibitor, a BCL2 inhibitor, or a BCR pathway inhibitor.

**Table 4: Additional molecularly defined subgroups for GOYA**

| **COODC Subtype** | | **GCB** | **ABC** | **UNCLASSIFIED** |
|---|---|---|---|---|
| Schmitz MCD-like | 12.4 | 2.1 | 34.4 | 6.8 |
| Schmitz BN2-like | 14.4 | 13.8 | 12.6 | 22.0 |
| Schmitz EZB-like | 19.2 | 31.1 | 0.7 | 8.5 |
| Schmitz N1-like | 2.4 | 1.0 | 4.0 | 5.1 |
| Multiple_Schmitz | 6.2 | 5.5 | 7.9 | 5.1 |
| Chapuy C1-like | 3.2 | 3.8 | 2.0 | 3.4 |
| Chapuy C2-like | 1.8 | 1.0 | 2.6 | 3.4 |
| Chapuy C3-like | 29.3 | 40.8 | 9.9 | 22.0 |
| Chapuy C4-like | 2.6 | 3.1 | 1.3 | 3.4 |
| Chapuy C5-like | 9.0 | 2.1 | 22.5 | 8.5 |
| Multiple Chapuy | 17.0 | 12.5 | 27.8 | 11.9 |
| BCR-dependent (CD79B+) | 9.4 | 1.7 | 24.5 | 8.5 |
| Double Hit | 1.4 | 2.1 | 0.0 | 1.7 |
| TP53 WT | 78.8 | 77.5 | 86.1 | 66.1 |
| BCL2 alteration | 23.2 | 33.6 | 9.9 | 6.8 |
| EZH2 alteration | 10.0 | 15.9 | 1.3 | 3.4 |
| Targetable | 85.0 | 86.2 | 88.7 | 69.5 |
| B2M alteration | 15.8 | 19.0 | 11.3 | 11.9 |
| CD274 amplification | 4.4 | 4.8 | 4.0 | 3.4 |
| CD58 alteration | 8.2 | 8.3 | 9.9 | 3.4 |
| CD70 alteration | 8.8 | 8.3 | 9.3 | 10.2 |
| CIITA alteration | 4.8 | 6.2 | 2.6 | 3.4 |
| Immune Evasion | 33.9 | 35.6 | 32.5 | 28.8 |

**Table 5: COO plus FM-Clinical**

| **COODC Subtype** | **ALL** | **GCB** | **ABC** | **UNCLASSIFIED** |
|---|---|---|---|---|
| Schmitz MCD-like | 13.0 | 1.1 | 38.4 | 9.8 |
| Schmitz BN2-like | 12.8 | 9.9 | 13.4 | 27.9 |
| Schmitz EZB-like | 29.7 | 47.4 | 2.3 | 4.9 |
| Schmitz N1-like | 2.6 | 0.9 | 5.8 | 3.3 |
| Multiple_Schmitz | 7.0 | 7.1 | 8.1 | 3.3 |
| Chapuy C1-like | 2.2 | 1.7 | 2.3 | 4.9 |
| Chapuy C2-like | 4.1 | 2.6 | 7.0 | 4.9 |
| Chapuy C3-like | 29.9 | 42.9 | 9.9 | 11.5 |
| Chapuy C4-like | 1.9 | 1.4 | 2.9 | 1.6 |
| Chapuy C5-like | 10.3 | 2.8 | 26.7 | 6.6 |
| Multiple Chapuy | 23.8 | 22.7 | 27.3 | 19.7 |
| BCR-dependent (CD79B+) | 8.0 | 1.4 | 22.7 | 4.9 |
| Double Hit | 1.7 | 2.8 | 0.0 | 0.0 |
| TP53 WT | 62.7 | 61.1 | 68.0 | 57.4 |
| BCL2 alteration | 32.8 | 50.3 | 5.8 | 8.2 |
| EZH2 alteration | 13.0 | 20.2 | 2.3 | 1.6 |
| Targetable | 80.7 | 86.4 | 75.0 | 63.9 |
| B2M alteration | 15.9 | 21.0 | 7.0 | 11.5 |
| CD274 amplification | 2.7 | 2.0 | 4.7 | 1.6 |
| CD58 alteration | 9.4 | 8.2 | 13.4 | 4.9 |
| CD70 alteration | 6.8 | 8.0 | 6.4 | 1.6 |
| CIITA alteration | 5.3 | 6.8 | 3.5 | 1.6 |
| Immune Evasion | 31.1 | 34.1 | 29.1 | 19.7 |

## Claims

1. A method of determining the cell of origin (COO) of a diffuse large B Cell lymphoma (DLBCL) of a patient diagnosed with DLBCL, the method comprising:
a. performing DNA sequencing on DNA from a sample acquired from the patient to acquire a list of genomic features associated with the sample,
b. applying, by a computer system, a pre-defined cell of origin DNA classification model to the list of genomic features to calculate a probability score, wherein the cell of origin DNA classification model is described by a set of weighted DNA-based features and is trained to identify activated B cell (ABC) COO and germinal center B Cell (GCB) COO samples using DNA-based features, wherein the set of weighted DNA-based features include chromosome arm-level copy number and zygosity metrics, gene-level features and one or more frequency of alteration class features, and
c. classifying, by the computer system, the patient as having a germinal center B cell (GCB) COO when the probability score is below a first pre-defined cutoff, and classifying, by the computer system, the patient as having an activated B cell (ABC) COO when the probability score is above or equal to a second predefined cutoff, wherein the first pre-defined cutoff is at or below the second pre-defined cutoff.

2. The method of claim 1, wherein the first pre-defined cutoff is between 0.2-0.3, and the second pre-defined cutoff is between 0.4-0.6.

3. The method of claim 1 or claim 2, wherein no RNA analysis is performed, and/or wherein RNA analysis does not contribute to the determination of the COO, or wherein the DNA comprises DNA obtained by subjecting the sample to reverse transcription of mRNA.

4. The method of any one of claims 1-3, wherein no immunohistochemistry (IHC) analysis is performed, and/or wherein IHC analysis does not contribute to the determination of the COO.

5. The method of any of claims 1-4, wherein alterations in the BCL2, EZH2, and TNSFRSF14 genes are detected, and/or wherein alterations in the chromosome 3q copy number, MYD88, and CD79B genes are detected, and/or wherein alterations in the NOTCH1, NOTCH2, and BCL6 genes are detected, and/or wherein an IGH:BCL2 rearrangement, a CREBBP alteration, and a TNFRSF14 alteration are detected, and/or wherein detection of a CDKN2A/B deletion, a CD79B alteration at amino acid 196, and an MYD88 alteration at amino acid 265 are detected; optionally wherein detection of alterations in the BCL2, EZH2, and TNSFRSF14 genes using the COODC predicts the COO of the DLBCL is a germinal center B Cell (GCB), and/or wherein detection of alterations in the chromosome 3q copy number, MYD88, and CD79B genes using the COODC predicts the COO of the DLBCL is an activated B Cell (ABC), and/or wherein detection of alterations in the NOTCH1, NOTCH2, and BCL6 genes using the COODC predicts the COO of the DLBCL is an activated B Cell (ABC), and/or wherein detection of an IGH:BCL2 rearrangement, a CREBBP alteration, and a TNFRSF14 alteration predicts the COO of the DLBCL is a GCB, and/or wherein detection of a CDKN2A/B deletion, a CD79B alteration at amino acid 196, and an MYD88 alteration at amino acid 265 predicts the COO of the DLBCL is an ABC.

6. The method of any preceding claim, further comprising classifying a patient having a predictor score above or equal to the first pre-defined cutoff and below the second pre-defined cutoff as unclassified.

7. The method of any of claims 1-6, wherein the patient is identified as having a GCB COO and selected for administration of a therapy that is effective intreating GCB COO, or wherein the patient is identified as having an ABC COO patient and selected for administration of a therapy that is effective for ABC COO.

8. The method of any of claims 1-7, wherein the sample is a clinical sample.

9. The method of claim 8, wherein the clinical sample is selected from the group consisting of a tumor biopsy, blood, bone marrow aspirate, cell-free DNA, circulating tumor DNA, and circulating tumor cells.

10. The method of claim 9, wherein the clinical sample comprises cell-free DNA.

11. The method of claim 9, wherein the clinical sample is a tumor biopsy preserved as a formalin-fixed paraffin-embedded (FFPE) preparation.

12. The method of any preceding claim, wherein the cell of origin DNA classification model was developed using penalized logistic regression.

13. The method of any preceding claim, wherein the set of features include one or more features selected from Table 1.

14. A computer system for determining the cell of origin (COO) of a diffuse large B Cell lymphoma (DLBCL) of a patient diagnosed with DLBCL, the computer system configured to perform a method comprising:
a. obtaining a list of genomic features associated with a sample from the patient acquired by performing DNA sequencing on DNA from the sample,
b. applying a pre-defined cell of origin DNA classification model to the list of genomic features to calculate a probability score, wherein the cell of origin DNA classification model is described by a set of weighted DNA-based features and is trained to identify activated B cell (ABC) COO and germinal center B Cell (GCB) COO samples using the set of weighted DNA-based features, wherein the set of weighted DNA-based features include chromosome arm-level copy number and zygosity metrics, gene-level features and one or more frequency of alteration class features, and
c. classifying the patient as having a germinal center B cell (GCB) COO when the probability score is below a first pre-defined cutoff, and classifying, by the computer system, the patient as having an activated B cell (ABC) COO when the probability score is above or equal to a second predefined cutoff, wherein the first pre-defined cutoff is at or below the second pre-defined cutoff.

15. The computer system of claim 14, wherein the computer system is configured to perform a method that has the features of any of claims 2 to 13.

## Patentansprüche

1. Verfahren zur Bestimmung der Ursprungszelle (COO) eines diffusen großzelligen B-Zell-Lymphoms (DLBCL) eines Patienten, bei dem DLBCL diagnostiziert wurde, wobei das Verfahren Folgendes umfasst:
a. Durchführen einer DNA-Sequenzierung an DNA von einer Probe, die von dem Patienten erhalten wurde, um eine Liste von genomischen Merkmalen zu erhalten, die der Probe zugeordnet sind,
b. Anwenden, mittels eines Computersystems, eines vordefinierten Ursprungszellen-DNA-Klassifizierungsmodells auf die Liste von genomischen Merkmalen, um einen Wahrscheinlichkeitswert zu berechnen, wobei das Ursprungszellen-DNA-Klassifizierungsmodell durch einen Satz von gewichteten Merkmalen auf DNA-Basis beschrieben wird und trainiert ist, um aktivierte B-Zell- (ABC-) COO- und Keimzentrum-B-Zell- (GCB-) COO-Proben anhand von Merkmalen auf DNA-Basis zu identifizieren, wobei der Satz von gewichteten Merkmalen auf DNA-Basis Kopienzahl und Zygositätsmetriken auf Chromosomenarm-Ebene, Merkmale auf Gen-Ebene und ein oder mehrere Merkmale bezüglich der Klasse der Häufigkeit von Veränderungen umfasst, und
c. Klassifizieren, mittels des Computersystems, des Patienten als eine Keimbahnzentrum-B-Zell- (GCB-) COO aufweisend, wenn der Wahrscheinlichkeitswert niedriger als ein erster vordefinierter Cutoff ist, und Klassifizieren, mittels des Computersystems, des Patienten als eine aktivierte B-Zell- (ABC-) COO aufweisend, wenn der Wahrscheinlichkeitswert gleich wie oder höher als ein zweiter vordefinierter Cutoff ist, wobei der erste vordefinierte Cutoff gleich wie oder kleiner als der zweite vordefinierte Cutoff ist.

2. Verfahren nach Anspruch 1, wobei der erste vordefinierte Cutoff zwischen 0,2 bis 0,3 liegt und der zweite vordefinierte Cutoff zwischen 0,4 bis 0,6 liegt.

3. Verfahren nach Anspruch 1 oder Anspruch 2, wobei keine RNA-Analyse durchgeführt wird und/oder wobei eine RNA-Analyse nicht zur Bestimmung der COO beiträgt oder wobei die DNA eine DNA umfasst, die durch Aussetzen der Probe gegenüber einer Umkehrtranskription von mRNA erhalten wird.

4. Verfahren nach einem der Ansprüche 1 bis 3, wobei keine immunhistochemische (IHC-) Analyse durchgeführt wird und/oder wobei eine IHC-Analyse nicht zur Bestimmung der COO beiträgt.

5. Verfahren nach einem der Ansprüche 1 bis 4, wobei Veränderungen im BCL3-, EZH2- und TNSFRSF14-Gen detektiert werden und/oder wobei Veränderungen in der Chromosom-3q-Kopienzahl oder im MYD88- und CD79B-Gen detektiert werden und/oder wobei Veränderungen im NOTCH1-, NOTCH2- und BCL6-Gen detektiert werden und/oder wobei eine IGH:BCL2-Umordnung, eine CREBBP-Veränderung und eine TNFRSF14-Veränderung detektiert werden und/oder wobei die Detektion einer CDKN2A/B-Deletion, einer CD79B-Veränderung bei Aminosäure 196 und einer MYD88-Veränderung bei Aminosäure 265 detektiert werden; wobei gegebenenfalls die Detektion von Veränderungen im BCL2-, EZH2- und TNSFRSF14-Gen unter Verwendung der COODC vorhersagt, dass die COO der DLBCL eine Keimbahn-B-Zelle (GCB) ist, und/oder wobei die Detektion von Veränderungen in der Chromosom-3q-Kopienzahl und im MYD88- und CD79B-Gen unter Verwendung der COODC vorhersagt, dass die COO der DLBCL eine aktivierte B-Zelle (ABC) ist, und/oder wobei die Detektion von Veränderungen im NOTCH1-, NOTCH2- und BCL6-Gen unter Verwendung der COODC vorhersagt, dass die COO der DLBCL eine aktivierte B-Zelle (ABC) ist, und/oder wobei die Detektion einer IGH:BCL2-Umordnung, einer CREBBP-Veränderung und einer TNFRSF14-Veränderung vorhersagt, dass die COO der DLBCL eine GCB ist, und/oder wobei die Detektion einer CDKN2A/B-Deletion, einer CD79B-Veränderung bei Aminosäure 196 und einer MYD88-Veränderung bei Aminosäure 265 vorhersagt, dass die COO der DLBCL eine ABC ist.

6. Verfahren nach einem der vorangegangenen Ansprüche, das weiters das Klassifizieren eines Patienten mit einem Vorhersagewert höher als oder gleich wie der erste vordefinierte Cutoff und kleiner als der zweite vordefinierte Cutoff als unklassifiziert umfasst.

7. Verfahren nach einem der Ansprüche 1 bis 6, wobei der Patient als eine GCB-COO aufweisend identifiziert wird und zur Verabreichung einer Therapie ausgewählt wird, die wirksam zur Behandlung von GCB-COO ist, oder wobei der Patient als ABC-COO-Patient identifiziert wird und zur Verabreichung einer Therapie ausgewählt wird, die wirksam für ABC-COO ist.

8. Verfahren nach einem der Ansprüche 1 bis 7, wobei die Probe eine klinische Probe ist.

9. Verfahren nach Anspruch 8, wobei die klinische Probe aus der aus einer Tumorbiopsie, Blut, einem Knochenmarkaspirat, zellfreier DNA, zirkulierender DNA eines Tumors und zirkulierenden Tumorzellen bestehenden Gruppe ausgewählt ist.

10. Verfahren nach Anspruch 9, wobei die klinische Probe zellfreie DNA umfasst.

11. Verfahren nach Anspruch 9, wobei die klinische Probe eine Tumorbiopsie ist, die als in Formalin fixiertes, in Paraffin eingebettetes (FFPE) Präparat konserviert ist.

12. Verfahren nach einem der vorangegangenen Ansprüche, wobei das Ursprungszellen-DNA-Klassifizierungsmodell unter Verwendung von penalisierter logistischer Regression entwickelt wurde.

13. Verfahren nach einem der vorangegangenen Ansprüche, wobei der Satz von Merkmalen ein oder mehrere aus Tabelle 1 ausgewählte Merkmale umfasst.

14. Computersystem zur Bestimmung der Ursprungszelle (COO) eines diffusen großzelligen B-Zell-Lymphoms (DLBCL) eines Patienten, bei dem DLBCL diagnostiziert wurde, wobei das Computersystem ausgelegt ist, um ein Verfahren auszuführen, das Folgendes umfasst:
a. Erlangen einer Liste von genomischen Merkmalen, die einer Probe vom Patienten zugeordnet sind, die durch Durchführen einer DNA-Sequenzierung an DNA von der Probe erhalten wurde,
b. Anwenden eines vordefinierten Ursprungszellen-DNA-Klassifizierungsmodells auf die Liste von genomischen Merkmalen, um einen Wahrscheinlichkeitswert zu berechnen, wobei das Ursprungszellen-DNA-Klassifizierungsmodell durch einen Satz von gewichteten Merkmalen auf DNA-Basis beschrieben wird und trainiert ist, um aktivierte B-Zell- (ABC-) COO- und Keimzentrum-B-Zell- (GCB-) COO-Proben unter Verwendung des gewichteten Satzes von Merkmalen auf DNA-Basis zu identifizieren, wobei der Satz von gewichteten Merkmalen auf DNA-Basis Kopienzahl und Zygositätsmetriken auf Chromosomenarm-Ebene, Merkmale auf Gen-Ebene und eine oder mehrere Merkmale bezüglich der Klasse der Häufigkeit von Veränderungen umfasst, und
c. Klassifizieren des Patienten als eine Keimbahnzentrum-B-Zell- (GCB-) COO aufweisend, wenn der Wahrscheinlichkeitswert niedriger als ein erster vordefinierter Cutoff ist, und Klassifizieren, mittels des Computersystems, des Patienten als eine aktivierte B-Zell-(ABC-) COO aufweisend, wenn der Wahrscheinlichkeitswert gleich wie oder höher als ein zweiter vordefinierter Cutoff ist, wobei der erste vordefinierte Cutoff gleich wie oder kleiner als der zweite vordefinierte Cutoff ist.

15. Computersystem nach Anspruch 14, wobei das Computersystem ausgelegt ist, um ein Verfahren auszuführen, das Merkmale nach einem der Ansprüche 2 bis 13 aufweist.

## Revendications

1. Procédé de détermination de la cellule d'origine (COO) d'un lymphome diffus à grandes cellules B (DLBCL) d'un patient diagnostiqué avec le DLBCL, le procédé comprenant les étapes consistant à :
a. effectuer un séquençage d'ADN sur de l'ADN provenant d'un échantillon acquis auprès du patient pour acquérir une liste de caractéristiques génomiques associées à l'échantillon,
b. appliquer, par l'intermédiaire d'un système informatique, une cellule prédéfinie de modèle de classification d'ADN d'origine à la liste de caractéristiques génomiques pour calculer un score de probabilité, dans lequel la cellule de modèle de classification d'ADN d'origine est décrite par un ensemble de caractéristiques pondérées basées sur de l'ADN et est formé pour identifier des échantillons de COO de cellule B activée (ABC) et de COO de cellule B de centre germinatif (GCB) en utilisant les caractéristiques basées sur de l'ADN, dans lequel l'ensemble de caractéristiques pondérées basées sur de l'ADN inclut le nombre de copies de niveau de bras chromosomique et des métriques de zygosité, des caractéristiques de niveau gène et une ou plusieurs fréquences de caractéristiques de classe d'altération, et
c. classer, par l'intermédiaire du système informatique, le patient comme présentant une COO de cellule B de centre germinatif (GCB) lorsque le score de probabilité est inférieur à un premier seuil prédéfini, et classer, par l'intermédiaire du système informatique, le patient comme présentant une COO de cellule B activée (ABC) lorsque le score de probabilité est supérieur ou égal à un second seuil prédéfini, dans lequel le premier seuil prédéfini est égal ou inférieur au second seuil prédéfini.

2. Procédé selon la revendication 1, dans lequel le premier seuil prédéfini est compris entre 0,2 et 0,3, et le second seuil prédéfini est compris entre 0,4 et 0,6.

3. Procédé selon la revendication 1 ou la revendication 2, dans lequel aucune analyse d'ARN n'est effectuée, et/ou dans lequel l'analyse d'ARN ne contribue pas à la détermination de la COO, ou dans lequel l'ADN comprend de l'ADN obtenu en soumettant l'échantillon à une transcription inverse d'ARNm.

4. Procédé selon l'une quelconque des revendications 1 à 3, dans lequel aucune analyse immunohistochimique (IHC) n'est effectuée, et/ou dans lequel l'analyse IHC ne contribue pas à la détermination de la COO.

5. Procédé selon l'une quelconque des revendications 1 à 4, dans lequel des altérations des gènes BCL2, EZH2 et TNSFRSF14 sont détectées, et/ou dans lequel des altérations du nombre de copies du chromosome 3q, des gènes MYD88 et CD79B sont détectées, et/ou dans lequel des altérations des gènes NOTCH1, NOTCH2 et BCL6 sont détectées, et/ou dans lequel un réagencement IGH:BCL2, une altération de CREBBP et une altération de TNFRSF14 sont détectées, et/ou dans lequel la détection d'une délétion de CDKN2A/B, d'une altération de CD79B au niveau de l'acide aminé 196, et d'une altération de MYD88 au niveau de l'acide aminé 265 sont détectées ; facultativement, dans lequel la détection d'altérations des gènes BCL2, EZH2 et TNSFRSF14 en utilisant la COODC prédit que le COO du DLBCL est une cellule B de centre germinatif (GCB), et/ou dans lequel la détection d'altérations du nombre de copies du chromosome 3q, des gènes MYD88 et CD79B en utilisant la COODC prédit que la COO du DLBCL est une cellule B activée (ABC), et/ou dans lequel la détection d'altérations des gènes NOTCH1, NOTCH2 et BCL6 en utilisant la COODC prédit que la COO du DLBCL est un lymphocyte B activé (ABC), et/ou dans lequel la détection d'un réagencement IGH:BCL2, d'une altération de CREBBP et d'une altération de TNFRSF14 prédit que la COO du DLBCL est une GCB, et/ou dans lequel la détection d'une délétion de CDKN2A/B, d'une altération de CD79B au niveau de l'acide aminé 196 et d'une altération de MYD88 au niveau de l'acide aminé 265 prédit que la COO du DLBCL est une ABC.

6. Procédé selon l'une quelconque des revendications précédentes, comprenant en outre la classification d'un patient présentant un score de prédiction supérieur ou égal au premier seuil prédéfini et inférieur au second seuil prédéfini comme non classé.

7. Procédé selon l'une quelconque des revendications 1 à 6, dans lequel le patient est identifié comme présentant une GCB COO et choisi pour l'administration d'un traitement qui est efficace pour traiter la COO GCB, ou dans lequel le patient est identifié comme présentant un patient COO ABC et choisi pour l'administration d'un traitement qui est efficace pour la COO ABC.

8. Procédé selon l'une quelconque des revendications 1 à 7, dans lequel l'échantillon est un échantillon clinique.

9. Procédé selon la revendication 8, dans lequel l'échantillon clinique est choisi dans le groupe constitué d'une biopsie tumorale, de sang, d'aspirat de moelle osseuse, d'ADN acellulaire, d'ADN tumoral circulant et de cellules tumorales circulantes.

10. Procédé selon la revendication 9, dans lequel l'échantillon clinique comprend de l'ADN acellulaire.

11. Procédé selon la revendication 9, dans lequel l'échantillon clinique est une biopsie tumorale conservée sous la forme d'une préparation fixée au formol et incluse en paraffine (FFPE).

12. Procédé selon l'une quelconque des revendications précédentes, dans lequel le modèle de classification d'ADN de cellule d'origine a été développé en utilisant une régression logistique pénalisée.

13. Procédé selon l'une quelconque des revendications précédentes, dans lequel l'ensemble de caractéristiques inclut une ou plusieurs caractéristiques choisies dans le Tableau 1.

14. Système informatique destiné à déterminer la cellule d'origine (COO) d'un lymphome diffus à grandes cellules B (DLBCL) d'un patient diagnostiqué avec un DLBCL, le système informatique étant configuré pour exécuter un procédé comprenant les étapes consistant à :
a. obtenir une liste de caractéristiques génomiques associées à un échantillon du patient acquises en effectuant un séquençage d'ADN sur l'ADN provenant de l'échantillon,
b. appliquer une cellule prédéfinie de modèle de classification d'AN d'origine à la liste de caractéristiques génomiques pour calculer un score de probabilité, dans lequel la cellule de modèle de classification d'ADN d'origine est décrite par un ensemble de caractéristiques pondérées basées sur de l'ADN et est formée pour identifier les échantillons de COO de cellule B activée (ABC) et de COO de cellule B de centre germinatif (GCB) en utilisant l'ensemble de caractéristiques pondérées basées sur de l'ADN, dans lequel l'ensemble de caractéristiques pondérées basées sur de l'ADN inclut le nombre de copies au niveau du bras chromosomique et des métriques de zygosité, des caractéristiques de niveau gène et une ou plusieurs caractéristiques de classe de fréquence d'altération, et
c. classer le patient comme présentant une COO de cellule B de centre germinatif (GCB) lorsque le score de probabilité est inférieur à un premier seuil prédéfini, et classer, par l'intermédiaire du système informatique, le patient comme présentant une COO de cellules B activée (ABC) lorsque le score de probabilité est supérieur ou égal à un second seuil prédéfini, dans lequel le premier seuil prédéfini est égal ou inférieur au second seuil prédéfini.

15. Système informatique selon la revendication 14, dans lequel le système informatique est configuré pour exécuter un procédé qui présente les caractéristiques de l'une quelconque des revendications 2 à 13.
